# EUROPEAN PATENT APPLICATION

(11) **EP 4 772 646 A1**
(43) Date of publication of application: **08.07.2026**
(21) Application number: 24858011.0
(22) Date of filing: 28.06.2024
(51) Int. Cl.: C12N 15/82, C12N 15/53, A01H 3/00, A01H 5/00, A01H 4/00

(54) **PLANT CULTIVATION METHOD, USE AND PRODUCT**

(30) Priority: 31.08.2023 CN 202311114382
(71) Applicant: Epiplant Co. Ltd., Beijing 100000 (CN)
(72) Inventor: JIA, Guifang, Beijing 100000 (CN); LI, Ji, Beijing 100000 (CN); LI, Weifeng, Beijing 100000 (CN)
(74) Representative: Adares PartGmbB
(86) International application number: PCT/CN2024/102215
(87) International publication number: WO 2025/044467

(57) **Abstract**

Provided is a plant cultivation method, the use and a product. The plant cultivation method comprises introducing an inducing medium into an initial plant and subsequently cultivating the plant to obtain a target plant which contains no inducing medium and has optimized traits compared with the initial plant. The inducing medium can induce demethylation of 6-methylated bases in plant RNA. The plant cultivation method can be used for obtaining plants which has optimized traits and contains no initially introduced inducing medium.

## Description

### Technical Field

The present invention relates to the field of plant cultivation, specifically to a plant cultivation method, use, the obtained target plant, and product.

### Background Technology

Methods of artificial intervention in plant cultivation typically encompass various approaches, such as genetic engineering, cell engineering, and microbial engineering, among others. To obtain new plant varieties, the aforementioned methods are commonly employed. The new plant varieties here include the entire plant, plant organs (such as flowers, fruits, seeds, roots, stems, leaves, etc.), plant tissues, plant cells, and other parts that can be derived from plants.

The newly obtained plant varieties are typically expected to exhibit more optimized traits, such as optimized growth, increased yield, optimized biomass, enhanced structure, or optimized cell division, among others. Among these optimized traits, increased yield can be achieved through various phenotypic factors, such as larger seed size or weight per seed/thousand seeds, increased tiller number in the root system, and other factors that influence plant yield, especially the increase in yield of the desired parts of the plant.

The aforementioned expectations are currently typically achieved through certain modification methods, such as altering the plant's DNA or histones to modify its traits. Prior to this invention, there have been approaches regarding the demethylation of plant RNA to achieve the purpose of optimizing plant traits.

### Summary of the Invention

The aforementioned approaches can indeed be used to obtain plants with optimized traits, particularly increased yield. However, plants obtained through these approaches still contain the initially introduced inducing medium, and even in the selection targets or criteria applied during plant selection, plants that contain the inducing medium and exhibit optimized traits are still adopted as the reference standard. Neverthless, it has been found by the applicant's research that plants with optimized traits can still be obtained even in the absence of the inducing medium. Moreover, the trait optimization degree achieved is not inferior to that of plants containing such inducing media.

Accordingly, the present invention provides a plant cultivation method by which plants exhibiting optimized traits can be obtained free of any introduced inducing medium. The provided specific technical solution is as follows:
A plant cultivation method, characterized by introducing an inducing medium into an initial plant and obtaining, through cultivation, a target plant that contains no inducing medium and exhibits optimized traits compared with the initial plant, wherein the inducing medium is capable of inducing demethylation of 6-methylated bases in plant RNA.

Optionally, the target plant has at least one optimized trait compared to the initial plant, with an optimization degree Y.

Optionally, the inducing medium is transiently introduced into the initial plant, such that the target plant, which contains no inducing medium and exhibits optimized traits compared with the initial plant, is obtained directly through cultivation.

Optionally, the inducing medium is transiently introduced into protoplasts of the initial plant.

Optionally, the inducing medium is introduced into the initial plant to obtain an intermediate plant, and the intermediate plant is subjected to progeny cultivation so as to obtain a target plant that contains no inducing medium and exhibits optimized traits compared with the initial plant.

Optionally, the intermediate plant contains an inducing medium.

Optionally, the target plant has at least one optimized trait compared with the initial plant, with an optimization degree Y.

Optionally, the intermediate plant has at least one optimized trait compared with the initial plant, with an optimization degree X.

Optionally, Y represents an increase ratio relative to the initial plant during the same period, wherein Y ≥ 0.20; Y ≥ 0.50; Y ≥ 1.00; preferably Y ≥ 2.00; preferably Y ≥ 3.00; and preferably Y ≥ 4.00.

Optionally, the trait optimization includes an increase in yield.

Optionally, the trait optimization includes an increase in the yield of plant organs.

Optionally, the trait optimization includes at least one of the following: an increase in the volume, quantity, or weight of plant organs, or an increase in the number of tillers.

Optionally, the inducing medium is capable of inducing demethylation of 6-methyladenine in plant RNA.

Optionally, the inducing medium is introduced in an amount excessive relative to the initial plant.

Optionally, the inducing medium is nucleic acid molecule and/or polypeptide, or its homolog, functional variant, or conjugate.

Optionally, when the inducing medium is nucleic acid molecule, or its homolog, functional variant, or conjugate, the target plant contains neither the inducing medium nor polypeptide expressed therefrom; when the inducing medium is polypeptide, or its homolog, functional variant, or conjugate, the target plant does not contain the inducing medium.

Optionally, the inducing medium is selected from RNA m6A demethylase and nucleic acid encoding it.

Optionally, the inducing medium is selected from at least one of FTO nucleic acid molecule and/or FTO polypeptide, or its homolog, functional variant, or conjugate.

Optionally, the FTO in the inducing medium is derived from vertebrates, invertebrates, algae, or their orthologs or paralogs.

Optionally, the inducing medium corresponds to the sequences shown in Sequence Listing Seq. ID No. 1 to Seq. ID No. 15.

Optionally, the initial plant is selected from at least one of food crops, feed crops, fiber crops, oil crops, sugar crops, beverage crops, spice crops, condiment crops, medicinal crops, dye crops, ornamental crops, fruit crops, and vegetable crops; preferably selected from at least one of Rapeseed plant, tomato, lettuce, and beet; or preferably selected from at least one of rice, corn, soybean plant, potato, wheat, millet, sugarcane, sorghum, and cassava; or preferably selected from at least one of tobacco, alfalfa, rubber grass, cotton, flax, sunflower, camelina, nutsedge, cannabis, and poplar.

Optionally, the initial plant part subjected to the introducing treatment includes at least one of plant organ, plant tissue, and plant cell.

Optionally, the initial plant part subjected to the introducing treatment is selected from meristematic tissues.

Optionally, the inducing medium is introduced into at least one of the nucleus and the cytoplasm of the initial plant.

Optionally, the introducing method includes introducing a vector carrying an inducing medium into the initial plant.

Optionally, the progeny cultivation method includes at least one of natural genetic screening removal, hybrid genetic screening removal, and active removal.

This invention also provides the use of the aforementioned plant cultivation method for the production of plants having optimized traits and free of the inducing medium.

This invention also provides a target plant obtained by the plant cultivation method described above, including at least one of a whole living plant, plant organ, plant tissue, or plant cell.

This invention also provides a product derived from the target plant subjected to inactivation.

### Illustration of the Attached Figure

Figure 1 schematically illustrates the demethylation of N6-methyladenine.
Figure 2 depicts the PCR-generated electrophoresis profile of plant samples.

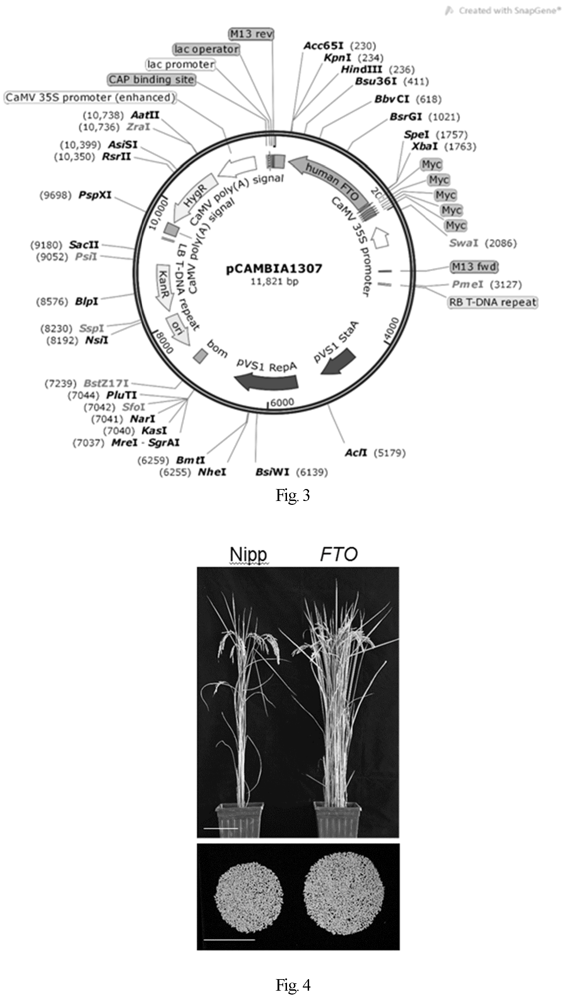

Figure 3 is an example from Embodiment 2, showing the transfer of the pCAMBIA1307 vector carrying the FTO gene and the hygromycin resistance gene (Hygromycin) into Agrobacterium tumefaciens LBA4404.
Figure 4 is a comparative illustration of the whole plant and rice grains between the initial plant (Nipp) and the intermediate plant (FTO), using rice as an Embodiment 2.

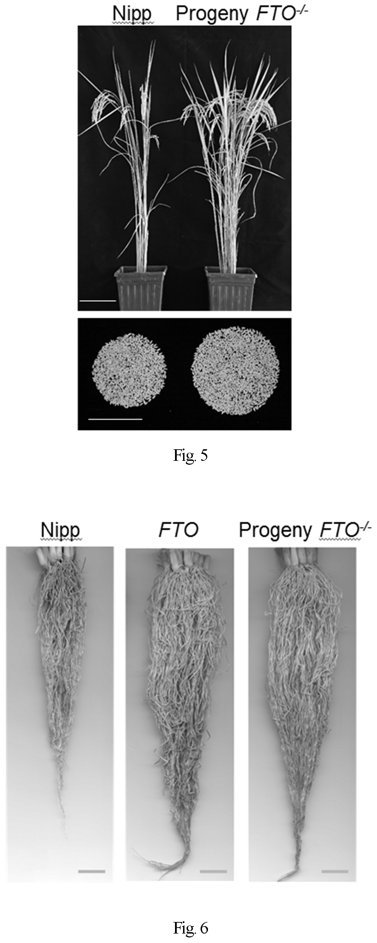

Figure 5 is a comparative illustration of the whole plant and rice between the initial plant (Nipp) and the target plant (Progeny FTO), using rice as an Embodiment 2.
Figure 6 is a comparative illustration of the root systems of the initial plant (Nipp), the intermediate plant (FTO), and the target plant (Progeny FTO), using rice as an Embodiment 2.

### Specific Implementation Method

In order to enable the person skilled in the art to better understand the technical solution of the present invention, the following provides a further detailed description of the present invention in conjunction with specific embodiments.

Through research for this invention, it has been found that during plant growth and development, specific enzymes can promote methylation of RNA bases. For example, methyltransferase protein MTA can catalyze N6-methylation of adenine bases in RNA, where "6-" refers to the sixth N counted from the N in the adenine base connected to the RNA sequence, either in forward or reverse order. After methylation, the RNA, through subsequent processes such as expression, or the products derived therefrom, can promote plant growth and development. Experimental evidence has shown that removal of such methylating enzymes adversely affects plant growth and development-for instance, plants may fail to produce viable seeds, or seeds may not germinate normally. However, it has been further found that partial methyl removal from methylated RNA enhances the ability of the RNA or its products to promote plant growth and development, thereby facilitating trait optimization. Such demethylation can be achieved using an appropriate inducing medium.

Conventionally, to obtain plants with optimized traits, plants retaining the inducing medium is typically selected in order to maintain the optimized traits. However, the applicant of this invention has unexpectedly found that plants subjected to RNA demethylation via a suitable inducing medium can retain their optimized traits even in the absence of the inducing medium. Moreover, these optimized traits can, in some cases, be stably inherited.

This invention study found that by introducing certain inducing medium capable of promoting RNA demethylation into plants, the plants' own traits can be optimized, and plants with optimized traits can be obtained free of inducing medium, particularly resulting in an increase in plant yield.

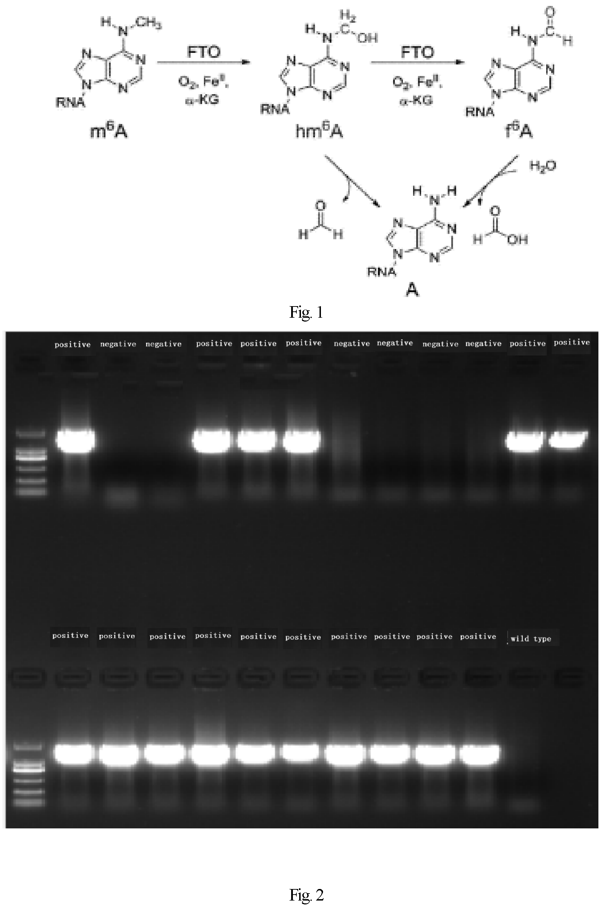

Such type of inducing medium for RNA demethylation primarily targets RNA with already methylated bases. Such inducing medium is mostly related to demethylases, such as FTO. The chemical mechanism of inducing is illustrated in Figure 1, taking the demethylation process of 6-methyladenine as an example. Studies have revealed that although the specific RNA sequences demethylated by such inducing medium is not yet clear, it is certain that such inducing medium can indeed demethylate RNA with methylated bases, thereby obtaining plants with optimized traits. More advantageously, plants with optimized traits that do not contain such inducing medium can also be obtained.

An embodiment of this invention provides a method for plant cultivation, which includes introducing inducing medium into the initial plant and obtaining a target plant which contains no inducing medium and has optimized traits compared to the initial plant; inducing medium can induce demethylation of 6-methylated bases in plant RNA.

The term "plant" used herein specifically refers to the whole plant or any of its active parts, including the entire plant, its progenitors and descendants, as well as plant organs such as seeds, branches, stems, leaves, roots, flowers, fruits, etc. The term "plant" also includes plant cells, suspension cultures, callus tissues, embryos, meristematic regions, gametophytes, sporophytes, pollen, and microspores, and may extend to harvestable parts of the plant of this invention, such as, but not limited to, the plant body and its descendants, or other obtainable active parts of the plant. The "plant" described in this invention is not limited to specific species, including, for example, herbaceous plants, woody plants, algae, ferns, and all plants belonging to the superfamily of the plant kingdom, preferably monocotyledons or dicotyledons.

The "plants" required for cultivating in this invention, as previously mentioned, can be selected from various plants, including those chosen from the following usage classifications: food crops (such as rice, corn, soybean plant, etc.), feed crops (such as alfalfa, etc.), fiber crops (such as cotton, cannabis, etc.), oil crops (such as sesame, peanuts, etc.), sugar crops (such as sugarcane, beets, etc.), beverage crops, spice crops, condiment crops, medicinal crops (such as ginseng, ganoderma, fritillaria, etc.), dye crops, ornamental crops, fruit crops, vegetable crops, etc.; or selected from any specific plant, for example, preferably selected from at least one of rapeseed plant (Brassica napus L.), tomato (Lycopersicon esculentum Mill.), lettuce (Lactuca sativa L. var. ramosa Hort.), beet (Beta vulgaris L.); or preferably selected from at least one of rice (Oryza sativa L.), com (Zea mays L.), soybean plant (Glycine max (Linn.) Merr.), potato (Solanum tuberosum), wheat (Triticum aestivum L.), millet (Setaria italica var. germanica (Mill.) Schred.), sugarcane (Saccharum officinarum), sorghum (Sorghum bicolor (L.) Moench), cassava (Manihot esculenta Crantz); or preferably selected from at least one of tobacco (Nicotiana tabacum L.), alfalfa (Medicago Sativa Linn), rubber grass (Taraxacum kok-saghyz Rodin), cotton (Gossypium spp), flax (Linum usitatissimum L.), sunflower (Helianthus annuus L.), camelina (Camelina sativa (L.) Crantz), nutsedge (Cyperus esculentus L.), cannabis (Cannabis sativa L.), poplar (Populus L.).

The term "initial plant" used herein refers to plants that have not been processed using the method described in this invention, and its species and structure may specifically encompass any of the aforementioned plants. The source of the "initial plant" can be either wild or artificially cultivated plants.

The term "target plant" used herein refers to the plant that has been treated by the method of this invention and is expected to be obtained, the type and structure of which can also encompass any of the aforementioned plants.

The term " a product derived from the... plant subjected to...treatment " used herein specifically refers to the products derived from the aforementioned "plants" after inactivation treatment, which are inactive; not limited to various fields, such as industrial, medicinal, edible, etc., nor limited to specific component products, such as dried granules, powder, oil, fat, fatty acids, starch, or protein, etc., and also not limited to specific product forms, such as solid, liquid, gaseous, or mixed states, etc.

The term "increased yield" as defined herein refers to the increase in weight of a specific harvestable part compared to the initial plant. For example, in the case of rice, the increase in the weight of rice grains is considered the increased yield, and for potatoes, the increase in the weight of the tubers is considered the increased yield, and so forth.

The term "increased biomass" as defined herein refers to the weight added to the entire plant, excluding the yield increase mentioned as above; for example, in the case of rice, the increased weight of the remaining parts, excluding the rice grains, constitutes the increased biomass.

The yield and biomass used herein are measured based on the dry weight of plants during the same period.

The implementation of the method of this invention provides plants with increased growth rates. Therefore, this invention provides a method for increasing plant growth rates, which includes introducing an incuding medium, which can promote RNA demethylation, into the plant to obtain the target plant free of the incuding medium.

Therefore, the present invention provides a method for increasing plant yield, comprising introducing an inducing medium into an initial plant and obtaining a target plant that is free of the inducing medium and exhibits an increased yield compared to the initial plant, wherein the inducing medium is capable of inducing RNA demethylation in the plant.

The traits of plants can be adjusted and optimized by introducing inducing medium for RNA demethylation. Optionally, when using homolog, functional variant/conjugate of inducing medium, the traits of plants can also be adjusted and optimized.

The term "inducing medium" as defined herein refers to a medium capable of inducing demethylation of RNA in plants. "Inducing" can refer to catalyzing or participating in any reaction, preferably as a catalytic action. Such mediums can currently be demethylases, such as FTO, among others. Furthermore, "RNA" includes one or more types of RNA in plants, such as ribosomal RNA (rRNA), messenger RNA (mRNA), transfer RNA (tRNA), etc.; "demethylation" refers to the methyl removal from already methylated RNA, which can occur at one or more sites on the RNA, such as demethylation of already methylated bases in RNA, for example, demethylation at its 1-methylated base, or demethylation at its 6-methylated base. Here, 1- and 6- define the positions of methylation, which can be, for example, the N starting position of the base connecting RNA as the 1-position, counting clockwise or counterclockwise to the 6-position. Here, "base" can be common bases in nucleic acid molecule, such as adenine (A), cytosine (C), guanine (G), etc.

The source of the "inducing medium" can be any one or more sources, such as from animals or microorganisms; more preferably, inducing medium may originate from vertebrates or algae, preferably from humans or green algae. The "source" as defined herein can be directly from vertebrates or algae, or indirectly from vertebrates or algae, such as from orthologs or paralogs of vertebrates or algae, preferably from orthologs or paralogs of humans or green algae.

The term "paralogous" involves gene duplication within the genome of a certain species, resulting in paralogous genes.

The term "orthologous" refers to homologous genes in different organisms that result from speciation.

The term "homology" herein refers to homologous sequence with equivalent function to the described inducing medium sequence; the term "functional variant/conjugate" herein refers to variants/conjugates of inducing medium with equivalent function obtained through mutation, conjugation, or other means.

The term "introduction" herein encompasses one or more aspects, such as introduction methods, introduction parts, introduction quantities, etc.

The inducing medium may be introduced via various routes. For example, a vector carrying an inducing medium may be used to infect a plant, the inducing medium may be inserted into the plant's DNA, or a vector carrying an inducing medium may be delivered into plant cells. The part subject to introduction is not particularly limited and may include, for example, the cell nucleus or the cytoplasm. In addition, the introduction may be transient or long-term, and may be at a low or excessive level, depending on the characteristics of the plant and the nature of the inducing medium.

The term "free of the inducing medium" herein refers to the target plant obtained that at least does not contain the introduced inducing medium.

For example, when inducing medium is nucleic acid molecule, its homolog, or its functional variant, the target plant does not contain inducing medium and the polypeptide expressed therefrom.

When inducing medium is polypeptide, its homolog, or its functional variants, the target plant does not contain inducing medium.

More specifically, this invention provides a method for cultivating plants, which includes the following steps:
An inducing medium is introduced into the initial plant to obtain an intermediate plant; and progeny thereof are obtained to produce a target plant free of the inducing medium and having optimized traits as compared with the initial plant.

This invention obtains the target plant, which does not contain inducing medium and has optimized traits compared to the initial plant, either directly or indirectly. Direct acquisition can be, for example, the transient introduction of inducing medium into the initial plant, especially the transient introduction into the protoplast cells of the initial plant. Indirect acquisition involves obtaining the second plant (target plant) through an intermediate plant, which is the first plant obtained after the introduction of inducing medium, and then cultivating its progeny. In both cases, the RNA demethylation of the plant is affected. The difference lies in whether an intermediate plant is involved. Similarly, the terms "intermediate plant" and "target plant" herein are structurally or categorically the same as the aforementioned plants.

The "inducing medium selected from RNA m6A demethylase and its encoding nucleic acid" described herein refers to the nucleic acid that can be obtained through the amino acid sequence of the RNA m6A demethylase of this invention, using various nucleic acids capable of encoding its amino acid sequence obtained by conventional reverse engineering in the field, or nucleic acids directly obtained from the source of RNA m6A demethylase.

The term "intermediate plant" herein can refer to any subsequent generation of the initial plant. The "intermediate plant" may contain inducing medium.

The "target plant" described herein can be any subsequent generation of the initial plant, wherein, when no intermediate plant is produced, the target plant is directly cultivated from the initial plant. When an intermediate plant is produced, the target plant is obtained from its progeny through cultivating the intermediate plant.

The method of the present invention is not intended to specifically limit the numerical extent of trait optimization, and may provide a qualitative rather than a quantitative description. In this invention method, any improvement or beneficial increase in plant traits is considered trait optimization. Furthermore, the degree of trait optimization defined herein as X, Y can also be defined by specific numerical values, all of which are based on the comparison with the same trait of the initial plant, such as an increase in seed quantity X, Y, or an increase in total seed weight X, Y.

The degree of trait optimization Y of the target plant can be the same as the degree of trait optimization X of the intermediate plant, not exceeding X, or even exceeding X. If calculated by the increase multiple, Y is the increase multiple compared to the initial plant at the same period, Y≥0.20; Y≥0.50; Y≥1.00; preferably Y≥2.00; preferably Y≥3.00; preferably Y≥4.00. Here, the increase multiple can be, for example, the weight increase multiple or others.

This invention method may select inducing medium as nucleic acid molecule and/or polypeptide, its homolog, or its functional variant/conjugate.

The term also refers to inducing medium that can be nucleic acid molecule or polypeptide, and can also be a substance mixed with nucleic acid molecule and polypeptide; furthermore, it refers to paralogs or orthologs with the aforementioned homology to nucleic acid molecule or polypeptide, or it may refer to the corresponding vectors, hybrids, or substances obtained through fusion. Specifically, the existing demethylation inducing medium can be one or more of the subsequent sequences in the Sequence Listing. It should be understood that inducing medium is not limited to the sequences listed in the Sequence Listing.

"Nucleic acid molecule and/or polypeptide, its homolog or its functional variant" may be any natural or synthetic substances.

The term "hybridization" as defined herein refers to the process of complementary nucleotide sequences, which are essentially homologous, annealing to each other. The hybridization process can occur entirely in solution, meaning that the complementary nucleic acids are both in solution. The hybridization process can also proceed in such a manner that one of the complementary nucleic acids is fixed to a substrate such as magnetic beads, agarose beads, or any other resin. Furthermore, the hybridization process can proceed in such a manner that one of the complementary nucleic acids is fixed to a solid-phase support such as a nitrocellulose membrane or nylon membrane, or fixed to a support such as silica glass through techniques such as photolithography. To enable hybridization to occur, nucleic acid molecule is typically thermally or chemically denatured to separate the double strands into two single strands and/or to remove hairpins or other secondary structures from single-stranded nucleic acids. The stringency of hybridization is influenced by conditions such as temperature, salt concentration, and the composition of the hybridization buffer.

Nucleic acid molecules or their variants may originate from any natural or artificial source. Nucleic acids/genes or their variants can be derived from microbial sources such as bacteria, yeast, or fungi, or from plant, algae, or animal (including human) sources. They can be modified from their natural form in terms of composition and/or genomic environment through rigorous artificial manipulation. The nucleic acids are preferably derived from algae, particularly from sources such as green algae.

The term "homolog" encompasses two special forms of homology, namely orthologous sequences and paralogous sequences, which involve evolutionary concepts used to describe genetic relationships.

The homologous substance can also be in the form of a protein "insertion variant," where one or more amino acid residues are introduced into predetermined positions within the protein. Such insertions may include fusion at the amino terminus and/or carboxyl terminus, as well as insertion within the sequence of single or multiple amino acids. Typically, insertions within the amino acid sequence will be smaller than fusions at the amino or carboxyl terminus, approximately ranging from 1 to 10 residues. Examples of fusion proteins or peptides at the amino or carboxyl terminus include binding domains or activation domains of transcription activators used in yeast two-hybrid systems, phage coat proteins, (histidine)6-tags, glutathione S-transferase tags, protein A, maltose-binding protein, dihydrofolate reductase, Tag·100 epitope, c-myc epitope, FLAG epitope, lacZ, CMP (calmodulin-binding peptide), HA epitope, C protein epitope, and VSV epitope.

Functional variant applicable in the method of this invention may also comprises optional splice variants of nucleic acid molecule or genes. The term "optional splice variant" as used herein encompasses such variants of nucleic acid sequences in which selected introns and/or exons have been removed, replaced, or added. Such variants are those in which the biological activity of the protein remains unaffected, and they can be obtained by selectively retaining functional fragments of the polypeptide. These splice variants can be naturally occurring or artificially created. Methods for producing such splice variants are well known in the art.

The following will illustrate, through specific embodiments, the advantages of increased yield or enhanced root systems of the new plants obtained by the plant cultivation method of this invention.

### Embodiment 1: A method for inducing the production of target plants by co-cultivating inducing medium with protoplasts.

### 1. Expression and Purification of Inducing medium

Experimental Group: Clone inducing medium into the pET28a vector expressed in Escherichia coli to obtain the inducing medium comprising N-terminus His₆-tag fusion. Transform the cloned plasmid into competent Escherichia coli (E. coli) gold cells for cultivating. When the OD600 reaches 0.6-0.8, add 0.5mM IPTG for inducing, express at 16°C for 20 hours, and harvest the bacteria. Sonicate for 10 minutes, then perform high-speed centrifugation at 13000 rpm for 30 minutes. Use the supernatant for subsequent purification experiments. First, perform Ni column affinity purification, followed by cation exchange column, and finally size exclusion chromatography. Concentrate the collected protein to a higher concentration of 10~20 mg/mL and use polyacrylamide gel electrophoresis to detect the purity of the protein.

Control group: Except for not using inducing medium, all other steps and parameters are the same as those of the experimental group.

### 2. Preparation of Protoplasts

First method: Sterilize the seeds in 70% ethanol and 0.4% hypochlorite solution for 15 minutes, wash three times with distilled water, and sow on 1/2 MS solid medium. The seedlings are grown in a greenhouse at 25°C with a photoperiod of 16 hours light and 8 hours dark. To isolate protoplasts, incubate the leaves, stems, and sheaths of 14-day-old seedlings in an enzyme solution (1.0% cellulase R10, 0.5% macerozyme R10, 0.45M mannitol, 20mM MES [pH 5.7], CPW solution) at 25°C in the dark with shaking (40 rpm) for 12 hours, then dilute with an equal volume of W5 solution. After filtration, collect the protoplasts at the bottom of the tube by centrifugation at 100g for 5 minutes. Resuspend the protoplasts in CPW21S solution (CPW solution containing 21% [w/v] sucrose, pH 5.8), then collect the protoplasts at the bottom of the tube by centrifugation at 80g for 7 minutes. Resuspend the protoplasts in W5 solution and centrifuge at 70g for 5 minutes to collect the protoplasts at the bottom of the tube. Finally, resuspend the protoplasts in W5 solution and count using a hemocytometer under a microscope. Dilute the protoplasts to a density of 1×10⁶ protoplasts/ml in MMG solution (0.4M mannitol, 15mM MgCl₂, 4mM MES [pH5.7]). Suitable for plants such as Arabidopsis thaliana and rice.

Second method: Take plant leaves grown in B5 medium for 3 weeks and digest them in an enzyme solution (1% cellulose R10, 0.25% macerozyme R10, 0.5M mannitol, 8mM CaCl₂, 5mM MES [pH5.7], 0.1% BSA) at 25°C in the dark for 5 hours. Resuspend the protoplasts twice with an equal volume of W5 solution. To obtain intact protoplasts, place the protoplasts in W5 solution on an equal volume of21 % sucrose gradient and centrifuge at 50g for 5 minutes, collect the intact protoplasts and resuspend them in W5 solution. Before PEG-mediated transfection, stabilize the prepared protoplast solution at 4°C for at least 1 hour. Suitable for plants such as tobacco.

### 3. Protoplast Transfection

Prior to transfection, 1~5×10⁵ protoplasts are suspended in 200µL of MMG solution, gently mixed with 5~20µL of protein solution (10-60µg of inducing medium protein) (separately for control and experimental groups) and 210µL of freshly prepared PEG solution (40% [w/v] PEG4000, 0.2M mannitol, and 0.1M CaCl₂), and incubated in the dark at 25°C for 10 minutes. After incubation, slowly add 950µL of W5 solution (2mM MES [pH5.7], 154mM NaCl, 125mM CaCl₂, and 5mM KCl), and thoroughly mix the resulting solution by inverting the tube. Centrifuge at 100g for 3 minutes to collect the protoplasts, and gently resuspend them in 1ml of WI solution (0.5M mannitol, 20mM KCl, and 4mM MES [pH5.7]). Finally, transfer the protoplasts to a multi-well plate and culture them in the dark at 25°C for 24-48 hours.

### 4. Protoplast Regeneration

Resuspend the transfected protoplasts in 0.5×B5 supplemented medium (0.5×B5 medium, 375mg/L CaCl₂·2H₂O, 18.35mg/L NaFe-EDTA, 270mg/L sodium succinate, 103g/L sucrose, 0.2mg/L 2,4-dichlorophenoxyacetic acid (2,4-D), 0.3mg/L 6-benzylaminopurine (BAP), and 0.1g/L MES), and add an equal volume of 2.4% agarose solution to dilute the protoplasts to 2.5×10⁵ protoplasts/ml. Spread the agarose-embedded protoplasts in a 6-well plate, cover with 2ml of 0.5×B5 liquid medium, and incubate at 25°C in the dark. After 7 days, replace with fresh 2ml of 0.5 ×B5 liquid medium. Transfer the 6-well plate to light conditions (16 hours light [30 µ molm⁻²s⁻¹] and 8 hours dark) and incubate at 25°C. After 3 weeks of culture, transfer the microcalli, which have grown to a diameter of several millimeters, to MS regeneration medium containing 30g/L sucrose, 0.6% plant agar, 0.1mg/L α-naphthaleneacetic acid (NAA), and 0.5mg/L BAP. After approximately 4 weeks on the regeneration medium, the induction of new shoot tissue is observed.

### 5. Regeneration of roots from plant shoot tissue, transplantation, management

After cutting the newly generated shoot tissue and transferring it to rooting medium to form complete seedlings, transplant the surviving plants to the greenhouse for conventional fertilization and water management. Harvest the seeds of the transformed seedlings.

### 6. Obtaining genetically optimized plants free of inducing medium.

The harvested transformed seedlings are free of inducing medium and can be identified through genomic DNA PCR detection. These plants, which do not contain inducing medium, are planted in the field, and their yield is evaluated through at least two field experiments to screen for plants with increased yield, ultimately obtaining the target plants that do not contain inducing medium.

The following data is calculated based on the target plants from the experimental group and the control group during the maturity stage. The measured data refers to the aforementioned content, calculating the percentage increase in yield of different plants relative to the yield of the control group plants, denoted as Y. The yield is calculated based on the harvested products or root systems of different plants, including but not limited to roots, stems, leaves, flowers, fruits, and seeds.

The yield of the control group plant in this invention refers to the yield of the initial plant without the introduction of inducing medium, where Y represents the percentage data of increase or enhancement of various traits based on the initial plant, with the unit being %. The aforementioned data pertains to dry weight, specifically obtained by weighing after the process of deactivation in an oven at 105°C for 20 minutes, followed by drying in an oven at 80°C for 20 hours. In Embodiment 1, inducing medium used is polypeptide or nucleic acid molecule encoding it, with specific sequences that can be the same as those in the Sequence Listing SEQ ID NOs: 1~4 of the specification, or homologous sequences with equivalent functions, or variants/conjugates with equivalent functions. Inducing medium may be derived from vertebrates, invertebrates, or algae.

The following data is based on a single variable premise (i.e., introduction of inducing medium sequence - experimental group or non-introduction of inducing medium sequence - control group), while other factors affecting plant yield and root increase are artificially unified, such as soil environment, air environment, water quality, other introduced components, cultivating conditions and methods, cultivating time cycle, etc., all being the same; all substances or components used can be obtained through commercial purchase.

**Table 1-Inducing medium derived from Human (Homo sapiens) FTO (Experimental Group)**

| Plant | The average increase in yield by Y multiples. |
|---|---|
| Rice | The average weight of individual rice (grains) increased by 4.16 times compared to the control group during the same period. |
| Potato | The average weight of potato (tubers) per plant increased by 3.82 times compared to the control group during the same period. |
| Com | The average weight of individual com (kemels) increased by 4.10 times compared to the control group during the same period. |
| Soybean Plant | The average weight of individual soybean plant (seeds) increased by 4.00 times compared to the control group during the same period. |
| Tomato | The average weight of individual tomato (fruit) per plant increased by 3.81 times compared to the control group during the same period. |
| Wheat | The average weight of individual wheat (grains) increased by 3.76 times compared to the control group during the same period. |
| Sugarcane | The average weight of individual sugarcane stalks (stems) increased by 4.13 times compared to the control group during the same period. |
| Sorghum | The average weight of individual sorghum stalks (stems) increased by 3.57 times compared to the control group during the same period. |
| Rapeseed plant | The average weight of individual Rapeseed plant (stem and leaves) increased by 4.21 times compared to the control group during the same period. |
| Lettuce | The average weight of individual lettuce (leaves) increased by 2.74 times compared to the control group during the same period. |
| Beet | The average weight of individual beet plants (stems and leaves) increased by 2.81 times compared to the control group during the same period. |
| Tobacco | The average weight of individual tobacco (leaves) increased by 3.66 times compared to the control group during the same period. |
| Alfalfa | The average weight of individual alfalfa plants (stems and leaves) increased by 2.41 times compared to the control group during the same period. |
| Rubber Grass | The average weight of a single rubber plant (stem) increased by 3.21 times compared to the control group during the same period. |
| Cotton | The average weight of a single cotton boll (seed cotton) increased by 3.92 times compared to the control group during the same period. |
| Flax | The weight of individual flax (stems, leaves, seeds) increased by an average of 3.76 times compared to the control group during the same period. |
| Sunflower | The average weight of individual sunflower (fruit) increased by 3.85 times compared to the control group during the same period. |
| Camelina | The average weight of single camelina (seeds) increased by 4.07 times compared to the control group during the same period. |
| Nutsedge | The average weight of individual nutsedge (stem) increased by 2.81 times compared to the control group during the same period. |
| Cannabis | The average weight of a single cannabis plant (stem and leaves) increased by 2.97 times compared to the control group during the same period. |
| Poplar | The average weight of individual poplar (roots) increased by 3.12 times compared to the control group during the same period. |
| Cassava | The average weight of individual cassava (tuber) increased by 2.83 times compared to the control group during the same period. |

The above Table 1 may use the Sequence Listing SEQ ID NOs: 1 human FTO-polypeptide sequence or DNA encoding the polypeptide sequence for expression, or homologous sequences with equivalent function or variants/conjugates thereof with equivalent function.

**Table 2-Inducing medium Derived from Pig (Sus scrofa) FTO (Experimental Group)**

| Plant | The average increase in yield by Y multiples. |
|---|---|
| Rice | The average weight of individual rice plants (fruits) increased by 3.61 times compared to the control group during the same period. |
| Potato | The average weight of individual potato (tubers) per plant increased by 3.02 times compared to the control group during the same period. |
| Com | The average weight of individual com (kemels) increased by 3.20 times compared to the control group during the same period. |
| Soybean Plant | The average weight of individual soybean plant (seeds) increased by 3.07 times compared to the control group during the same period. |
| Tomato | The average weight of individual tomato fruits increased by 3.53 times compared to the control group during the same period. |
| Wheat | The average weight of individual wheat (grains) increased by 4.21 times compared to the control group during the same period. |
| Sugarcane | The average weight of a single sugarcane stalk (stem) increased by 4.10 times compared to the control group during the same period. |
| Sorghum | The average weight of a single sorghum stalk (stem) increased by 3.56 times compared to the control group during the same period. |
| Rapeseed plant | The average weight of individual Rapeseed plant (stems and leaves) increased by 2.40 times compared to the control group during the same period. |
| Lettuce | The average weight of individual lettuce (leaves) increased by 2.71 times compared to the control group during the same period. |
| Beetroot | The average weight of individual beet (stems and leaves) increased by 2.72 times compared to the control group during the same period. |
| Tobacco | The average weight of a single tobacco (leaf) increased by 2.53 times compared to the control group during the same period. |
| Alfalfa | The average weight of individual alfalfa plants (stems and leaves) increased by 2.65 times compared to the control group during the same period. |
| Rubber Grass | The average weight of individual rubber plant (stem) increased by 2.78 times compared to the control group during the same period. |
| Cotton | The average weight of cotton bolls (seed cotton) per plant increased by 3.61 times compared to the control group during the same period. |
| Flax | The average weight of individual flax (stems, leaves, seeds) increased by 4.00 times compared to the control group during the same period. |
| Sunflower | The average weight of a single sunflower (fruit) increased by 3.42 times compared to the control group during the same period. |
| Camelina | The average weight of individual camelina (seeds) increased by 3.25 times compared to the control group during the same period. |
| Nutsedge | The average weight of individual nutsedge (stem) increased by 2.73 times compared to the control group during the same period. |
| Cannabis | The average weight of a single cannabis plant (stems and leaves) increased by 2.94 times compared to the control group during the same period. |
| Poplar | The average weight of a single poplar (root) increased by 2.02 times compared to the control group during the same period. |
| Cassava | The average weight of a single cassava (tuber) increased by 2.75 times compared to the control group during the same period. |

The above Table 2 may use the porcine FTO-polypeptide sequence of Sequence Listing SEQ ID NOs:2 or DNA for expressing the polypeptide sequences, its functionally equivalent homologous sequences, or its functionally equivalent variants/conjugates.

**Table 3-Inducing medium Derived from Bovine (Bos taurus) FTO (Experimental Group)**

| Plant | The average increase in yield by Y multiples. |
|---|---|
| Rice | The average weight of individual rice (grains) increased by 3.71 times compared to the control group during the same period. |
| Potato | The average weight of individual potato (tubers) per plant increased by 2.79 times compared to the control group during the same period. |
| Com | The average weight of a single com (kemels) increased by 3.04 times compared to the control group during the same period. |
| Soybean Plant | The average weight of individual soybean plant (seeds) increased by 3.07 times compared to the control group during the same period. |
| Tomato | The average weight of a single tomato (fruit) increased by 3.49 times compared to the control group during the same period. |
| Wheat | The average weight of individual wheat plants (fruits) increased by 4.12 times compared to the control group during the same period. |
| Sugarcane | The average weight of a single sugarcane stalk (stem) increased by 4.06 times compared to the control group during the same period. |
| Sorghum | The average weight of individual sorghum stalks (stems) increased by 3.42 times compared to the control group during the same period. |
| Rapeseed plant | The average weight of individual Rapeseed plant (stem and leaves) increased by 2.36 times compared to the control group during the same period. |
| Lettuce | The average weight of individual lettuce (leaves) increased by 2.61 times compared to the control group during the same period. |
| Beetroot | The average weight of individual beet plants (stems and leaves) increased by 2.65 times compared to the control group during the same period. |
| Tobacco | The average weight of individual tobacco (leaves) increased by 2.42 times compared to the control group during the same period. |
| Alfalfa | The average weight of individual alfalfa plants (stems and leaves) increased by 2.57 times compared to the control group during the same period. |
| Rubber Grass | The average weight of individual rubber grass (stem) increased by 2.60 times compared to the control group during the same period. |
| Cotton | The weight of the cotton boll (seed cotton) per plant increased by an average of 3.53 times compared to the control group during the same period. |
| Flax | The average weight of individual flax (stems, leaves, seeds) increased by 3.96 times compared to the control group during the same period. |
| Sunflower | The average weight of a single sunflower (fruit) increased by 3.32 times compared to the control group during the same period. |
| Camelina | The average weight of individual camelina (seeds) increased by 3.15 times compared to the control group during the same period. |
| Nutsedge | The average weight of individual nutsedge (stem) increased by 2.74 times compared to the control group during the same period. |
| Cannabis | The average weight of a single cannabis plant (stems and leaves) increased by 2.83 times compared to the control group during the same period. |
| Poplar | The average weight of individual poplar (roots) increased by 1.97 times compared to the control group during the same period. |
| Cassava | The average weight of individual cassava tubers increased by 2.68 times compared to the control group during the same period. |

The above Table 3 may use the bovine FTO-polypeptide sequence of Sequence Listing SEQ ID NOs:3 or DNA for expressing the polypeptide sequence, its functionally equivalent homologous sequences, or its functionally equivalent variants/conjugates.

**Table 4 - Inducing medium derived from Green Alga (Ostreococcus lucimarinus) FTO (Experimental Group)**

| Plant | The average increase in yield by Y multiples. |
|---|---|
| Rice | The average weight of individual rice (grains) increased by 3.43 times compared to the control group during the same period. |
| Potato | The average weight of a single potato (tubers) per plant increased by 3.36 times compared to the control group during the same period. |
| Com | The average weight of a single com (kernels) increased by 3.32 times compared to the control group during the same period. |
| Soybean Plant | The average weight of individual soybean plant (seeds) increased by 3.38 times compared to the control group during the same period. |
| Tomato | The average weight of individual tomato fruits increased by 3.43 times compared to the control group during the same period. |
| Wheat | The average weight of a single wheat plant (fruit) increased by 3.18 times compared to the control group during the same period. |
| Sugarcane | The average weight of a single sugarcane stalk (stem) increased by 3.22 times compared to the control group during the same period. |
| Sorghum | The average weight of individual sorghum stalks (stems) increased by 3.41 times compared to the control group during the same period. |
| Rapeseed plant | The average weight of a single Rapeseed plant plant (stem and leaves) increased by 3.22 times compared to the control group during the same period. |
| Lettuce | The average weight of individual lettuce (leaves) increased by 3.37 times compared to the control group during the same period. |
| Beetroot | The average weight of individual beet (stems and leaves) increased by 3.12 times compared to the control group during the same period. |
| Tobacco | The average weight of individual tobacco (leaves) increased by 3.35 times compared to the control group during the same period. |
| Alfalfa | The average weight of individual alfalfa plants (stems and leaves) increased by 3.37 times compared to the control group during the same period. |
| Rubber Grass | The average weight of individual guayule plants (stems) increased by 3.35 times compared to the control group during the same period. |
| Cotton | The average weight of single cotton bolls (seed cotton) increased by 3.30 times compared to the control group during the same period. |
| Flax | The average weight of individual flax (stems, leaves, seeds) increased by 3.05 times compared to the control group during the same period. |
| Sunflower | The average weight of individual sunflower (fruit) increased by 3.43 times compared to the control group during the same period. |
| Camelina | The average weight of single camelina (seeds) increased by 3.05 times compared to the control group during the same period. |
| Nutsedge | The average weight of a single nutsedge (stem) increased by 3.31 times compared to the control group during the same period. |
| Cannabis | The average weight of individual cannabis plants (stems and leaves) increased by 3.10 times compared to the control group during the same period. |
| Poplar | The average weight of a single poplar tree (root) increased by 3.28 times compared to the control group during the same period. |
| Cassava | The average weight of single cassava (tuber) increased by 3.30 times compared to the control group during the same period. |

The above Table 4 may use the polypeptide sequence of Green Algae FTO-SEQ ID NOs:4 or the DNA for expressing the polypeptide sequence, its homologous sequences with equivalent function, or its variants/conjungates with equivalent function.

### Embodiment 2: A method for obtaining intermediate plants through Agrobacterium-mediated infection of callus tissue and screening target plants in the progeny.

### 1. Inducing callus tissue using plant seeds as test material.

### 1) Sterilization:

Take mature plant seeds, manually remove the shells, and select seeds that are plump, clean, and free of sterile spots. Place the seeds into a 100ml sterile beaker, pour in 70% alcohol for sterilization for 2 minutes; discard the alcohol, add 20% sodium hypochlorite (NaClO) solution, and soak for 30 minutes; discard the sodium hypochlorite solution, rinse the seeds 4-5 times with sterile distilled water, and finally soak the seeds in sterile distilled water for 30 minutes.

### 2) Induction Culture (requires aseptic operation):

After sterilizing the seeds, place them on sterile filter paper to absorb surface moisture, then transfer them into NB medium containing 2.0mg/L 2,4-D (pH 5.8), with 12-14 seeds per dish. To ensure the inducing rate, it is best to maintain the germination direction of the seeds parallel or slightly downward to the medium during inoculation, avoiding upward or vertically downward directions. After the operation, seal the petri dishes with sealing film and incubate them in a light incubator at 30°C with approximately 50% humidity. Conduct dark inducing culture for 20-30 days until obvious loose callus tissue appears, then proceed with subculture or preculture transformation.

### 3) Subculture (requires aseptic operation):

Open the petri dish on the ultra-clean workbench, use tweezers to select callus tissue that has naturally split, is growing vigorously, has a hard texture, a tender yellow color, and a particle diameter of approximately 3mm, and place it into NB medium containing 2.0mg/L 2,4-D and 0.5mg/L 6-BA (pH 5.8). Place 10 pieces of callus tissue in each dish and incubate in the dark at 30°C. If the callus tissue becomes severely softened, it is recommended to transfer it to light for subculture. The subculture duration is approximately 10 to 15 days (depending on the growth of the callus tissue, determine the next subculture time), with a total of two subcultures. Note: If a significant amount of water is found on the lid during the culture process in the petri dish, it is unsuitable and may lead to culture failure. The reason is that the bottom of the petri dish is heated, and the lid temperature is lower than the medium temperature, causing water vapor to condense on the petri dish lid

### 2. Agrobacterium Culture

Experimental group: Transfer the vector carrying an inducing medium and the hygromycin resistance gene Hygromycin into the parasitic fungus, for example, transfer the pCAMBIA 1307 vector carrying an inducing medium and the hygromycin resistance gene Hygromycin into Agrobacterium LBA4404, and inoculate it onto YEP solid medium containing 20 mg/L Rifampicin (Rit) and 50 mg/L Kanamycin (Kan).

Control Group: The vector containing the hygromycin resistance gene Hygromycin is introduced into the parasitic fungus, for example, the pCAMBIA 1307 vector containing the hygromycin resistance gene Hygromycin is introduced into Agrobacterium LBA4404, and inoculated onto YEP solid medium containing 20mg/L rifampicin (Rif) and 50mg/L kanamycin (Kan).

After culturing the control group and the experimental group at 28°C for 2 days, select Agrobacterium monoclonal colonies to perform colony PCR to verify whether inducing medium has been transferred into the Agrobacterium. Choose positive monoclonal colonies and culture them in 4ml YEP medium (containing 50 mg/L Kan and 20 mg/L Rif) at 28°C, shaking at 220 rpm for 20-36 hours until the bacterial suspension reaches an OD600 of 0.8-1.0.

### 3. Infection and co-cultivation

1) Centrifuge the prepared Agrobacterium suspension (separate control group and experimental group) at 4°C, 4000 rpm, for 10 minutes, and remove the supernatant. Use AAM medium containing 100 umol/L acetosyringone to make a suspension, adjusting the final concentration of the bacterial suspension to an OD600 of approximately 0.2.
2) Pick out the rice callus that has grown to a certain size and place it into the Agrobacterium suspension for infection for 20-30 minutes.
3) Take out the callus and place it on sterile filter paper to drain for 20-30 minutes, so as to avoid excessive growth of Agrobacterium during co-cultivating, which may cause excessive damage to the callus.
4) Place the callus tissue in NB medium containing 2.0mg/L 2,4-D and 100umol/L acetosyringone (pH 5.2), incubate in the dark at 25°C for 48~72 hours.
Note: Co-cultivating requires tracking and observation. If Agrobacterium becomes apparent, it indicates excessive infection, which may lead to bacterial growth during subsequent screening.

### 4. Screening of Resistant Callus

Take out the callus tissue and wash it 5-6 times with sterile water, continuously shaking during the process. Then place the callus tissue on sterilized filter paper to air dry, and evenly distribute it on NB medium containing 50mg/L hygromycin (pH 5.8) for the first round of selection screening. Incubate in the dark at 28°C, and if mold or Agrobacterium growth is observed, promptly transfer the infected callus tissue to a new selection plate.

After approximately 30 days of screening, once new callus tissue has grown, transfer it to fresh NB medium containing 50 mg/L hygromycin (pH 5.8) for further screening for 7 to 10 days. If significant growth is observed, it is considered positive callus tissue; if no growth occurs, even if browning or death does not appear, it may still be a false positive.

### 5. Inducing, differentiation, and rooting of positive callus.

Place the second round of screened, vigorously growing, yellow callus granules (ensuring that the callus tissue used for differentiation is flawless) onto NB medium (pH 5.8) containing 2.0 mg/L 6-BA, 0.5 mg/L kinetin, and 50 mg/L hygromycin. Place 2-3 positive clones per bottle, with a small amount of callus tissue for each clone. The callus tissue placed should be of high quality, regardless of quantity. Incubate in the dark at 27°C for 10 days, followed by light-induced differentiation for 10-20 days until green leaves emerge, after which rooting can proceed. Light intensity should be 4000lux, 14h/d.

Place each robust seedling differentiated from the clones into 1/2N6 medium (pH 5.8) containing 0.5mg/L naphthaleneacetic acid and 50mg/L hygromycin to induce rooting, with 2-3 seedlings per bottle. Cultivate under light at 27~30°C, with a light intensity of 4000lux for 14h/d. After 7~10 days, open the sealing film and add an appropriate amount of sterile water. Transplant after 2~3 days.

Note: During the differentiation process, callus should not be subcultured. The time from differentiation to transplantation for intermediate plants is approximately three months.

### 6. Acclimation and Transplantation of Intermediate Plants

Select the seedlings of intermediate plants with well-differentiated roots and stems/leaves (when the seedlings grow to the top of the test tube, the cover should be opened promptly), open the sealing film, add an appropriate amount of distilled water or sterile water (to prevent the culture medium from growing bacteria), acclimate the seedlings for about 3 days to a week, then wash off the agar, transplant them into soil pots in the greenhouse for growth, test, and obtain intermediate plants.

### 7. Verification of Intermediate Plant Testing

1) PCR Test: Design inducing medium primers, such as FTO-F: ATGAAGCGCACCCCGACTG; FTO-R: GGGTTTTGCTTCCAGAAGCTGA; PCR reaction program: 95°C for 5 minutes, 95°C for 15 seconds, 58°C for 15 seconds, 72°C for 30 seconds, 35 cycles, 72°C extension for 10 minutes, stored at 4°C. After the reaction is completed, the PCR product is analyzed by 1% agarose gel electrophoresis.
2) A method for rapid selection of intermediate plants under hygromycin treatment: Cut and collect fresh green leaves approximately 1cm long from the seedlings to be tested (with incisions at both ends), and lay them flat on the detection medium (0.7% agar, 1ml/L of 6-BA, 50mg/L of hygromycin) at 28°C, with a light/dark cycle of 16h/8h for 48h. Leaves that remain fresh green indicate positive plants, while negative seedlings show patchy necrosis on the leaves.

### 8. Evaluation of the Effects of Inducing medium in Intermediate Plants

The following data is obtained based on the plants in the middle of the experimental group and the control group during the maturity stage. The measured data refers to the aforementioned content, calculating the percentage increase in yield X of different plants relative to the yield of the control group plants. The yield is calculated based on the harvested products or roots of different plants, including but not limited to roots, stems, leaves, flowers, fruits, and seeds.

The yield of the control group plants in this invention refers to the yield of the initial plants without the introduction of inducing medium, whereas Y represents the percentage increase or enhancement of various traits based on the initial plants, with the unit being %. The aforementioned data pertains to weight as dry weight, specifically obtained by weighing after the initial plants are subjected to 105°C oven drying for 20 minutes and 80°C oven drying for 20 hours.

The following data is based on a single variable premise (i.e., introduction of inducing medium sequence - experimental group or non-introduction of inducing medium sequence - control group), while other factors affecting plant yield and root increase are artificially unified, such as soil environment, air environment, water quality, other introduced components, cultivating conditions and methods, cultivating time cycle, etc., all being the same; all substances or components used can be obtained through commercial purchase.

### 9. Obtaining target plants with genetic improvement free of inducing medium.

### 1) Obtained from intermediate plants.

In plants with heterozygous overexpression of inducing medium, when cells undergo meiosis to form gametes, inducing medium segregates along with the separation of homologous chromosomes, entering one of the gametes. After self-fertilization, it is independently inherited by the offspring through the gametes, resulting in gene segregation that produces progeny without inducing medium and progeny with overexpression of inducing medium. Plants without inducing medium and hygromycin gene in their genome can be screened and obtained through genomic DNA PCR detection. These plants, which do not contain inducing medium and hygromycin gene, are cultivated and evaluated for optimized traits through at least two experiments, selecting target plants with optimized traits. Ultimately, target plants with optimized traits that do not contain inducing medium and hygromycin gene in their genome are obtained.

### 2) Obtained from the hybrid separation of intermediate plants and wild types.

Crop hybridization involves the fertilization process where the pistil of one variety receives pollen from another variety to obtain hybrid seeds. For example, rice is a self-pollinating crop with both male and female organs on the same flower. It requires the removal of the male organs from the T3 generation of pure line strains that overexpress inducing medium, followed by artificial pollination with wild-type rice pollen to achieve hybridization and obtain hybrid seeds. The hybrid seeds are intermediate plants with heterozygous overexpression of inducing medium. Self-pollination will lead to gene segregation, resulting in progeny that do not contain inducing medium type and those that do. Screening can be conducted using genomic DNA PCR detection to obtain rice that does not contain inducing medium and hygromycin genes. These plants, which do not contain inducing medium and hygromycin genes, are cultivated and evaluated for optimized traits through at least two experiments. The target plants with optimized traits are selected, ultimately obtaining target plants with optimized traits that do not contain inducing medium and hygromycin genes in their genome.

### 3) Obtained through the natural loss in the generational inheritance process of intermediate plants.

In the process of crop reproduction and seed propagation, meiosis occurs to form reproductive cells, and the combination of sperm (pollen) and egg completes the fertilization process to form a zygote, which then develops into a seed. This process increases species diversity and promotes the evolution of species genotypes. During the successive propagation of seed plants, there may be a natural loss of introduced exogenous genes. Once the exogenous genes are lost, the target plants with optimized traits free of inducing medium is obtained. In the process of successive inheritance of overexpressed inducing medium strains, it is found through continuous generational genomic DNA PCR detection that exogenous genes in the genome are lost, meaning that the inducing medium and hygromycin genes cannot be detected. Based on existing experience, the loss of exogenous genes can be observed in the T2 generation. These plants, which do not contain the inducing medium and hygromycin genes, are cultivated and evaluated for their optimized traits through at least two experiments, screening for target plants with optimized traits, ultimately obtaining target plants with optimized traits whose genomes do not contain the inducing medium and hygromycin genes.

### Note: Method of genomic DNA PCR detection:

Design gene primers such as FTO-F: AGGAAGTTCATTTCATTTGGAGAGGAC; FTO-R: GGGTTTTGCTTCCAGAAGCTGA; the PCR reaction program is: 95°C for 5 minutes, 95°C for 15 seconds, 60°C for 120 seconds, 72°C for 30 seconds, 35 cycles, 72°C extension for 10 minutes, store at 4°C. After the reaction is completed, the PCR product is analyzed by 1% agarose gel electrophoresis.

Design hygromycin gene primers Hyg-F: CTTCTACACAGCCATCGGTC; Hyg-R: ACAATCCCACTATCCTTCGC. The PCR reaction program is as follows: 95°C for 5 minutes, 95°C for 15 seconds, 55°C for 120 seconds, 72°C for 30 seconds, 35 cycles, 72°C extension for 10 minutes, and storage at 4°C. After the reaction is complete, the PCR product is analyzed by 1% agarose gel electrophoresis.

The following are the specific operational methods:

### I. Plant DNA Extraction Method

Kit Name: DNAquick Plant System (Rapid Plant Genomic DNA Extraction System)
Brand: TIANGEN

1. Handling Materials:
   Take 100 mg of fresh plant tissue and place it into a 2 ml tube with a steel bead, or 10 mg into a well of a 96-well plate (reduce the extraction solution by half for the 96-well plate). Store in a -80°C freezer for more than half an hour. Shake in a grinder for 1 minute, add 400 µl of buffer FP1 and 6 µl of RNase A (10 mg/ml), vortex for 1 minute, and leave at room temperature for 10 minutes. Note: Due to the vast diversity of plant materials, the optimal amount of experimental material should be determined based on the differences in materials or different tissues of the same material.
2. Add 130 ul of buffer solution FP2, mix thoroughly, and vortex for 1 minute.
3. Centrifuge at 12,000 rpm (~13,400xg) for 5 minutes, and transfer the supernatant to a new centrifuge tube.
4. Optional Step: Centrifuge the supernatant again at 12,000 rpm (~13,400xg) for 5 minutes, and transfer the supernatant to a new centrifuge tube.
   Note: The purpose of this step is to remove precipitated impurities from the supematant, thereby increasing the purity of the extracted genomic DNA.
5. Add 0.7 times the volume of isopropanol to the supernatant, mix thoroughly, at which point flocculent genomic DNA will appear. (For example, add 350 ul of isopropanol to 500 ul of supernatant), centrifuge at 12,000 rpm (~13,400xg) for 2 minutes, discard the supernatant, and retain the precipitate.
6. Add 600 ul of 70% ethanol, vortex for 5 seconds, centrifuge at 12,000 rpm (~13,400xg) for 2 minutes, discard the supernatant.
7. Repeat step 6.
8. Open the lid and invert, allowing it to air dry completely at room temperature for 5-10 minutes to remove any residual ethanol.
   Note: The residue of ethanol may affect subsequent enzyme reactions (such as enzyme digestion, PCR, etc.) experiments.
9. Add 200ul of elution buffer TE, and dissolve the DNA in a 65-degree water bath for 10-60 minutes, inverting and mixing several times to aid dissolution, finally obtaining a DNA solution.

### II. PCR Reaction

1. Reagent
Specific Primer: FTO Primer
PCR Enzyme Mix: New Blue Dye High-Fidelity Taq Enzyme Mix, Brand: 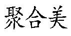
Primer: FTO Primer, Forward Primer FTO-F, Reverse Primer FTO-R

2. Configure the reaction system
Add each component sequentially into a 0.2ml centrifuge tube or the wells of a PCR plate.

| Reagent | Final concentration |
|---|---|
| Forward primer | 0.5ul |
| Reverse Primer | 0.5ul |
| Plant DNA | 3ul |
| PCR enzyme mix | 10ul |
| ddH₂O | 6ul |

3. After the amplification is completed according to the set time and temperature, which is shown below, perform electrophoresis identification.

Pre-denaturation at 95°C for 3 minutes, then proceed to the cycle amplification stage: 95°C for 30 seconds → 58°C for 30 seconds → 72°C for 20 seconds, cycle 30-35 times, maintain at 72°C for 5 minutes.

### III. Agarose Nucleic Acid Electrophoresis

1. Wash the apparatus used for electrophoresis with distilled water and set up the comb.
2. Prepare an appropriate concentration of 1.5% agarose gel. Accurately weigh 1.5g of agarose and add it to a conical flask, then add approximately 100ml of electrophoresis buffer (TAE).
3. After heating and melting in the microwave, take it out and let it cool down for a while before adding 10µl of dye (10000X), mix thoroughly, and then pour it into the electrophoresis tank.
4. At room temperature, solidify for approximately 40 minutes, carefully remove the comb, and place the gel in the electrophoresis tank to prepare for sample loading.
5. Add electrophoresis buffer to the electrophoresis tank, just enough to cover the surface of the gel, ensuring no air bubbles are present in the sample wells.
6. Prepare the sample before loading (in an eight-tube strip), add 5µl of sample and Marker, and use a pipette to slowly inject the mixed sample into the loading wells, ensuring no cross-infection between wells.
7. Connect the power supply according to the positive and negative poles (red positive, black negative), with a voltage of 40-60V and a duration of 30-40 minutes. The termination of electrophoresis can be determined based on the position of bromophenol blue.
8. After electrophoresis is completed, turn off the power, perform gel imaging observation, and compare with the marker to determine the fragment size.

### IV. Test Results:

As shown in the electrophoresis diagram in Figure 2: the presence of bands indicates the presence of the FTO gene, while the absence of bands indicates that the sample does not contain the FTO gene.

### 10. Evaluation of the traits of the target plant

The following data is obtained based on the target plants from the experimental group and the control group during the maturity stage. The measured data refers to the aforementioned content, calculating the percentage increase in yield of different plants relative to the yield of the control group plants, denoted as Y. The yield is calculated based on the harvested products or root systems of different plants, including but not limited to roots, stems, leaves, flowers, fruits, and seeds.

The yield of the control group plants in this invention refers to the yield of the initial plants without the introduction of inducing medium, whereas Y represents the percentage increase or enhancement of various traits based on the initial plants, with the unit being %. The aforementioned data pertains to weight as dry weight, specifically obtained by weighing after the initial plants are subjected to 105°C oven drying for 20 minutes and 80°C oven drying for 20 hours.

The following data is based on a single variable premise (i.e., introduction of inducing medium sequence - experimental group or non-introduction of inducing medium sequence - control group), while other factors affecting plant yield and root increase are artificially unified, such as soil environment, air environment, water quality, other introduced components, cultivating conditions and methods, cultivating time cycle, etc., all being the same; all substances or components used can be obtained through commercial purchase.

Embodiment 2 adopts nucleic acid molecule or polypeptide as inducing medium , with specific sequences that may use the same sequences as those listed in the specification sequence table SEQ ID NOs: 1~8 or SEQ ID NOs: 12~15, or homologous sequences with equivalent functions, or variants/conjugates with equivalent functions; inducing medium may be derived from vertebrates or algae.

The solution used in Embodiment 2 can be specifically exemplified as follows:
1. The formula for YEP liquid medium for Agrobacterium growth (amount per liter): Yeast extract 10g/L + Peptone 10g/L + NaCl 5g/L, pH 7.2, solid medium supplemented with 15g/L agar.
2. Agrobacterium resuspension solution AAM culture medium: 50ml 20×AA macronutrients, 10ml 100×FeEDTA, 10ml 100×B5 macronutrients, 10ml 100×B5 vitamins, 100ml 10×AA amino acids, 1ml 100mM acetosyringone, 68.5g sucrose, 36g glucose, 0.5g hydrolyzed casein, adjust volume to 1000ml, adjust pH to 5.2, sterilize through a 0.22mm cellulose acetate membrane.
3. 20×AA Major Elements: 59g KCl, 3g CaCl₂·2H₂O, 10g MgSO₄·7H₂O, and 3g NaH₂PO₄·H₂O, distilled water to a final volume of 1L, stored at 4°C.
4. 10×AA Amino Acids: 8.76 g Gin, 2.66 g Asp, 1.74 g Arg, and 75 mg Gly are diluted to 1L with distilled water, sterilized through a 0.22 mm cellulose acetate membrane, and stored at 4°C.
5. 100×B5 Vitamin: 10 g Inositol, 1 g Thiamine Hydrochloride, 100 mg Pyridoxine Hydrochloride, and 100 mg Niacin distilled water to a final volume of 1L, stored at 4°C.
6. 100×B5 Major Elements: 1.320 mg MnSO₄·4H₂O, 200 mg ZnSO₄·7H₂O, 2.5 mg CuSO₄·5H₂O, 25 mg Na₂MoO₄·2H₂O, 2.5 mg CoCl₂·6H₂O, 300 mg H₃BO₃, and 75 mg KI. Distilled water is used to make up to 1L, stored at 4°C.
7. NB Medium: N6 medium macronutrients and micronutrients, B5 medium organic elements, 300mg/L hydrolyzed casein, 500mg/L glutamine, 30g/L sucrose, and 8g/L agar.

### Embodiment 3: A method for obtaining intermediate plants through Agrobacterium mediated infection of stem tip tissues and screening target plants in the progeny.

### 1. Using mature plant embryos as experimental materials to induce callus tissue.

Soak mature seeds in clean water, changing the water every 1-2 hours. After 2 days, when the seeds begin to split and expose the white embryo, conduct germination and rooting at 37°C. Once the embryo grows to 1.5-2.0 cm, use a scalpel to remove the embryo and coleoptile from the stem apex and stem ring, exposing the apical meristem of the stem.

### 2. Agrobacterium Culture (same as Step 2 in Embodiment 2)

### 3. Infection and and co-cultivation

Place the processed plants into the AAM resuspension solution (see Step 3 of Embodiment 2), add 200l/L of the surfactant Silwet L-77, subject them to vacuum treatment at 1.5Kpa for 8 minutes, discard the resuspension solution, and place them on clean, moist perlite. Incubate in the dark at 28°C for 3 days.

### 4. Selection, transplantation, and management of intermediate plants.

After dark incubation, transfer the plants to the plant seedbed. At the three to four leaf stage, collect leaves from the transformed plants for PCR detection and identification of the transformed seedlings: design gene primers, for example, FTO-F: ATGAAGCGCACCCCGACTG; FTO-R: GGGTTTTGCTTCCAGAAGCTGA. The PCR reaction program is as follows: 95°C for 5 minutes, 95°C for 15 seconds, 58°C for 15 seconds, 72°C for 30 seconds, 35 cycles, 72°C extension for 10 minutes, and storage at 4°C. After the reaction is complete, analyze the PCR products using 1% agarose gel electrophoresis. Seven days later, transplant the surviving plants to the transgenic plant demonstration base greenhouse, with regular fertilization and water management. Harvest the seeds of the transformed seedlings.

### 5. Evaluation of the Traits of Intermediate Plants (Same as Step 8 of Embodiment 2)

### 6. Obtaining target plants with genetic improvement free of inducing medium.

The harvested transformed seedlings may undergo genetic segregation, capable of resulting in a type of progeny that do not contain the inducing medium and a type of progeny that overexpress the inducing medium. Plants that do not contain the inducing medium and hygromycin genes can be screened and obtained through genomic DNA PCR detection (as in Embodiment 2, Step 9). These plants, which do not contain the inducing medium and hygromycin genes, are then cultivated and evaluated for optimized traits through at least two experiments. The target plants with optimized traits are selected, ultimately obtaining target plants with optimized traits that do not contain the inducing medium and hygromycin genes in their genome.

The harvested transformed seedlings may naturally lose inducing medium. These plants, which do not contain inducing medium and hygromycin gene (as determined by genomic DNA PCR detection in the same manner as Step 9 of Embodiment 2), are planted and evaluated for optimized traits through at least two experiments. Target plants with optimized traits are screened, ultimately obtaining target plants with optimized traits whose genomes do not contain inducing medium and hygromycin gene.

### 7. Evaluation of Target Plant Traits (same as Step 10 in Embodiment 2)

### Embodiment 4: Method for obtaining intermediate plants through Agrobacterium mediated infection and inducing the formation of callus tissue, and then selecting target plants in the progeny (this method can be applied to any living plants to directly obtain target plants by inducing the formation of callus tissue).

### 1. Plant Cultivation

Plant mature seeds in nutrient soil, and grow them to maturity (approximately 63~66 days) in a greenhouse environment at 24°C with 16 hours of light and 8 hours of darkness.

### 2. Agrobacterium Culture

DRs (Growth Regulatory Elements) are a class of growth regulatory elements that induce the formation of meristem in plants, commonly used ones include BBM, ipt, ΔMP, STM, Wus2, etc.

Experimental Group: The vector carrying inducing medium, hygromycin resistance gene Hygromycin, and DRs is transferred into Agrobacterium GV3101. It is cultured overnight at 28°C for 12 hours in growth medium (10mM MES, pH5.6, 20µM acetosyringone, 50mg/L kanamycin, 50mg/L gentamicin), followed by centrifugation at 5000rpm for 10 minutes to collect the bacteria, which are then resuspended in infiltration medium (10mM MES, 150µM acetosyringone, 10mM MgCl₂) to achieve an OD600 value of 0.2~0.3. Prior to inoculation, the Agrobacterium mediated infection solution is incubated at room temperature (approximately 25°C) for 2~4 hours.

Control Group: The vector carrying the hygromycin resistance gene Hygromycin and DRs is transferred into Agrobacterium GV3101, cultured overnight at 28°C for 12 hours in growth medium (10mM MES, pH5.6, 20µM acetosyringone, 50mg/L kanamycin, 50mg/L gentamicin), then centrifuged at 5000rpm for 10 minutes to collect the bacterial cells, which are resuspended in infiltration medium (10mM MES, 150µM acetosyringone, 10mM MgCl2) to achieve an OD600 value of 0.2~0.3. Prior to inoculation, the Agrobacterium mediated infection solution is incubated at room temperature (approximately 25°C) for 2~4 hours.

### 3. Infection and Screening of Intermediate Plants

Remove visible branch meristems from all plants, leaving 2-3 nodes and supporting leaves. Immediately use a syringe and a 31G needle to inoculate the Agrobacterium mediated infection solution at the wound site. Observe bud formation at the cut site of the plant 38-48 days after inoculation. Each injection site with newly formed tissue or meristem is counted as a single event. Perform genomic PCR identification on the appearance of shoot tissue (see Step 7 of Embodiment 2) as an indicator of the presence and expression of transgenes.

### 4. Transplantation and Management of Intermediate Plants

After cutting the newly generated shoot tissue and transferring it to rooting medium to form complete seedlings, transplant the surviving plants to the greenhouse for conventional fertilization and water management. Harvest the seeds of the transformed seedlings.

### 5. Evaluation of the Traits of Intermediate Plants (same as Step 8 of Embodiment 2)

### 6. Obtaining the target plant with optimized traits free of inducing medium.

The harvested transformed seedlings may undergo genetic segregation, capable of resulting in a type of progeny that do not contain the inducing medium and a type of progeny that overexpress the inducing medium. Screening can be conducted through genomic DNA PCR detection to obtain plants whose genomes do not contain the inducing medium and hygromycin genes. These plants, which do not contain the inducing medium and hygromycin genes, are then cultivated and evaluated for optimized traits through at least two experiments. The target plants with optimized traits are selected, ultimately obtaining target plants with optimized traits whose genomes do not contain the inducing medium and hygromycin genes.

The harvested transformed seedlings may naturally lose the inducing medium. These plants, which do not contain the inducing medium and hygromycin gene, are cultivated and evaluated for their optimized traits through at least two experiments. Target plants with optimized traits are then selected, ultimately obtaining target plants with optimized traits whose genomes do not contain the inducing medium and hygromycin gene.

### 7. Evaluation of Target Plant Traits (same as Step 10 of Embodiment 2)

### Embodiment 5: A method for obtaining target plants by mechanical inoculation via PVX vector

### 1. Plant cultivation

Plant mature seeds in nutrient soil, and grow them to maturity (approximately 63~66 days) in a greenhouse environment at 24°C with 16 hours of light and 8 hours of darkness.

### 2. Agrobacterium Culture

Experimental Group: The vector pPZPVX carrying inducing medium was transferred into Agrobacterium C58Cl, cultured in Luria broth medium, and the bacterial cells were collected and suspended in infection buffer (10mM MES [pH 5.8], 10mM MgCl₂, 100µg/mL acetosyringone) to an OD600 value of 0.2~0.3. Prior to inoculation, the Agrobacterium mediated infection solution was incubated at room temperature (approximately 25°C) for 2~4 hours.

Control group: The vector pPZPVX free of inducing medium was transferred into Agrobacterium C58Cl, cultured in Luria broth medium, and then the bacterial cells were collected and suspended in the infection buffer (10mM MES [pH 5.8], 10mM MgCl₂, 100 µg/mL acetosyringone) to an OD600 value of 0.2~0.3. Before inoculation, the Agrobacterium mediated infection solution was incubated at room temperature (approximately 25°C) for 2~4 hours.

### 3. Infection and Screening of Intermediate Plants

Inject Agrobacterium carrying the plasmid expressing the inducing medium into tobacco leaves using a syringe. Seven days later, homogenize the inoculated leaves with 10mM NaPi buffer (pH 7.0) (verify the expression of inducing medium via western blot). After homogenization, centrifuge at 16,000g for 3 minutes and filter the supernatant using a 0.45µm filter. Apply the filtered supernatant along with carborundum (600 mesh) onto the fourth or fifth true leaf, gently rub by hand to mechanically inoculate PVX. After inoculation, place the plants under a 16-hour light/8-hour dark cycle at 16°C for growth, and observe the formation of new buds at the inoculation site 7 to 8 days later.

Note: Western blot detection of inducing medium expression: Mix the powdered leaf tissue after Infection with 1× loading buffer, heat at 95°C for 10 minutes, centrifuge at 15,000 rpm at room temperature for 10 minutes, and collect the supernatant. Load onto 10% SDS-PAGE, transfer the membrane after electrophoresis, block, and use inducing medium antibodies to detect the expression of the inducing medium.

### 4. Transplantation and Management of Intermediate Plants

After cutting the newly generated shoot tissue and transferring it to rooting medium to form complete seedlings, transplant the surviving plants to the greenhouse for conventional fertilization and water management. Harvest the seeds of the transformed seedlings.

### 5. Evaluation of the Traits of Intermediate Plants (Same as Step 8 of Embodiment 2)

### 6. Obtaining the target plant with optimized traits free of inducing medium.

The harvested transformed seedlings do not contain inducing medium and can be identified through genomic DNA PCR testing. These plants, which do not contain inducing medium, are cultivated and evaluated for optimized traits through at least two experiments. The target plants with optimized traits are screened, ultimately obtaining target plants with optimized traits that do not contain inducing medium and hygromycin genes in their genome.

### 7. Evaluation of Target Plant Traits (same as Step 10 of Embodiment 2)

### Embodiment 6: Method for Obtaining Target Plants through Haploid Induction-Edit (HI-Edit) Technology

Haploid inducing technology (HI) can induce haploids through appropriate modification of CENTROMERIC HISTONE H3 or knockout of MATRILINEAL, followed by chromosome doubling, thereby rapidly obtaining homozygous candidate materials and fixing crop genotypes, which is of significant importance in crop breeding practice. Haploid inducing-edit technology (HI-Edit) combines haploid inducing cultivating with gene editing technology, enabling direct improvement of a wide range of plants, including commercial varieties, in a short time, freeing from the limitations of existing gene editing systems imposed by species and genotypes. Since the chromosomes of the donor carrying gene-editing elements introduced through haploid-inducing lines are eliminated during the hybridization process, the resulting hybrids do not contain genetic information derived from the paternal parent, thereby eliminating the need for multiple backcrosses. Chromosome doubling of the target haploid plants alone can yield optimized diploid lines, shortening the cultivating cycle. It can improve target crops through distant hybridization, has a wide range of applications, and can obtain transgene-free lines.

### 1. Construct the inducing medium vector and introduce it into the non-haploid inducible inbred line NP2222 to obtain stable transformed plants.

Using 9-day-old immature embryos isolated from the self-crossing line NP2222, the isolated immature embryos are mixed with Agrobacterium strain LBA4404 containing a vector carring an inducing medium (experimental group) or Agrobacterium strain LBA4404 containing a vector without inducing medium (control group). The infected embryos are cultured on recovery medium and callus induction medium. Callus is selected on medium containing mannose, and resistant callus is regenerated into intermediate plants to obtain stably transformed plants.

### 2. Evaluation of the Traits of Intermediate Plants (same as Step 8 of Embodiment 2)

### 3. Select intermediate plants containing inducing medium to hybridize with the natural haploid inducing line RWKS, and select individuals containing inducing medium and homozygous matl mutation.

The natural haploid inducing line RWKS contains the haploid inducing inducer matl allele and the R1 color marker. After crossing RWKS with a plant line otaining the inducing medium, the progeny will exhibit trait segregation. Select the hybrid progeny containing the inducing medium, the haploid inducer matl allele from RWKS, and the R1 color marker, and perform self-pollination. From the F2 generation, select individuals containing the inducing medium and the homozygous matl mutation.

### 4. Screening and Obtaining of Target Plants

Cross the F2 plants homozygous for mat1 and inducing medium with various plant inbred lines again, and screen for haploid plants with the genotype MATL and not containing the inducing medium using the unique grain color marker of RWKS. Obtain the target plants through embryo rescue. The harvested modified plants not containing the inducing medium can be identified by genomic DNA PCR detection. These plants not containing the inducing medium is planted and evaluated for optimized traits through at least two experiments, screening for target plants with optimized traits. Ultimately, obtain target plants with optimized traits whose genomes do not contain the inducing medium and hygromycin gene.

### 5. Evaluation of Target Plant Traits (same as Step 10 in Embodiment 2)

The aforementioned Embodiments 2 to 6 all require obtaining the target plant through an intermediate plant, and all measure the degree of trait optimization. The following table data is presented using the data from Embodiment 2, and the data from the other Embodiments differ from that of Embodiment 2 by no more than ±5%. Therefore, further elaboration is not provided here.

**Table 5-Inducing medium derived from Human (Homo sapiens) FTO (Experimental Group)**

| Plant | The average increase in yield by X multiples. | The average increase in yield by Y multiples. |
|---|---|---|
| Rice | The average weight of a single rice plant's grain (fruit) increased by 4.15 times compared to the control group during the same period. | The average weight of individual rice (grains) increased by 4.05 times compared to the control group during the same period. |
| Potato | The average weight of individual potato (tubers) per plant increased by 3.83 times compared to the control group during the same period. | The average weight of a single potato (tuber) increased by 3.66 times compared to the control group during the same period. |
| Com | The average weight of a single com (kemels) increased by 4.12 times compared to the control group during the same period. | The average weight of a single com (kemels) increased by 3.27 times compared to the control group during the same period. |
| Soybean Plant | The average weight of individual soybean plant (seeds) increased by 4.00 times compared to the control group during the same period. | The average weight of individual soybean plant (seeds) increased by 3.54 times compared to the control group during the same period. |
| Tomato | The average weight of a single tomato (fruit) increased by 3.56 times compared to the control group during the same period. | The average weight of a single tomato (fruit) increased by 3.62 times compared to the control group during the same period. |
| Wheat | The average weight of individual wheat (grains) increased by 3.78 times compared to the control group during the same period. | The average weight of individual wheat (grains) increased by 4.21 times compared to the control group during the same period. |
| Sugarcane | The average weight of a single sugarcane stalk (stem) increased by 4.02 times compared to the control group during the same period. | The average weight of a single sugarcane stalk (stem) increased by 4.15 times compared to the control group during the same period. |
| Sorghum | The average weight of a single sorghum stalk (stem) increased by 3.52 compared to the control group during the same period. | The average weight of individual sorghum stalks (stems) increased by 3.57 times compared to the control group during the same period. |
| Rapeseed plant | The average weight of individual Rapeseed plant (stem and leaves) increased by 4.21 times compared to the control group during the same period. | The average weight of a single Rapeseed plant plant (stem and leaves) increased by 4.26 times compared to the control group during the same period. |
| Lettuce | The average weight of individual lettuce (leaves) increased by 2.73 times compared to the control group during the same period. | The average weight of individual lettuce (leaves) increased by 2.78 times compared to the control group during the same period. |
| Beetroot | The average weight of individual beet (stems and leaves) increased by 2.76 times compared to the control group during the same period. | The average weight of individual beet plants (stems and leaves) increased by 2.81 times compared to the control group during the same period. |
| Tobacco | The average weight of individual tobacco (leaves) increased by 3.65 times compared to the control group during the same period. | The average weight of individual tobacco (leaves) increased by 3.69 times compared to the control group during the same period. |
| Alfalfa | The average weight of individual alfalfa plants (stems and leaves) increased by 2.43 times compared to the control group during the same period. | The average weight of individual alfalfa plants (stems and leaves) increased by 2.71 times compared to the control group during the same period. |
| Rubber Grass | The average weight of individual rubber plant (stem) increased by 2.75 times compared to the control group during the same period. | The average weight of a single rubber plant (stem) increased by 2.82 times compared to the control group during the same period. |
| Cotton | The average weight of cotton bolls (seed cotton) per plant increased by 3.91 times compared to the control group during the same period. | The average weight of cotton bolls (seed cotton) per plant increased by 3.65 times compared to the control group during the same period. |
| Flax | The average weight of a single flax plant (stem, leaves, seeds) increased by 3.77 times compared to the control group during the same period. | The average weight of individual flax (stems, leaves, seeds) increased by 4.01 times compared to the control group during the same period. |
| Sunflower | The average weight of a single sunflower (fruit) increased by 3.88 times compared to the control group during the same period. | The average weight of a single sunflower (fruit) increased by 3.47 times compared to the control group during the same period. |
| Camelina | The average weight of individual camelina (seeds) increased by 4.09 times compared to the control group during the same period. | The average weight of individual camelina (seeds) increased by 4.02 times compared to the control group during the same period. |
| Nutsedge | The average weight of individual nutsedge (stem) increased by 2.75 times compared to the control group during the same period. | The average weight of individual nutsedge (stem) increased by 2.81 times compared to the control group during the same period. |
| Cannabis | The average weight of individual cannabis plants (stems and leaves) increased by 2.93 times compared to the control group during the same period. | The average weight of a single cannabis plant (stem and leaves) increased by 2.86 times compared to the control group during the same period. |
| Poplar | The average weight of individual poplar (roots) increased by 2.01 times compared to the control group during the same period. | The average weight of individual poplar (roots) increased by 2.05 times compared to the control group during the same period. |
| Cassava | The average weight of single cassava (tuber) increased by 2.79 times compared to the control group during the same period. | The average weight of individual cassava (tuber) increased by 2.83 times compared to the control group during the same period. |

The above Table 5 may use the polypeptide sequence of human FTO listed as SEQ ID NOs:1 or DNA for expressing the polypeptide sequence, its functionally equivalent homologous sequences, or its functionally equivalent variants/conjugates.

**Table 6-Inducing medium derived from Pig (Sus scrofa) FTO (Experimental Group)**

| Plant | The average increase in yield by X multiples. | The average increase in yield by Y multiples. |
|---|---|---|
| Rice | The average weight of individual rice (grains) increased by 3.62 times compared to the control group during the same period. | The average weight of single rice (grains) increased by 4.01 times compared to the control group during the same period. |
| Potato | The average weight of individual potato (tubers) per plant increased by 3.80 times compared to the control group during the same period. | The average weight of individual potato (tubers) per plant increased by 3.62 times compared to the control group during the same period. |
| Com | The average weight of individual com (kemels) increased by 4.10 times compared to the control group during the same period. | The average weight of individual com (kemels) increased by 3.24 times compared to the control group during the same period. |
| Soybean Plant | The average weight of individual soybean plant (seeds) increased by 3.95 times compared to the control group during the same period. | The average weight of a single soybean (seed) relative to the control group during the same period increased by 3.53 times. |
| Tomato | The average weight of individual tomato (fruit) increased by 3.52 times compared to the control group during the same period. | The average weight of individual tomato (fruit) increased by 3.59 times compared to the control group during the same period. |
| Wheat | The average weight of a single wheat plant (fruit) increased by 3.71 times compared to the control group during the same period. | The average weight of single wheat (grains) per plant increased by 4.17 times compared to the control group during the same period. |
| Sugarcane | The average weight of a single sugarcane stalk (stem) increased by 4.00 times compared to the control group during the same period. | The average weight of individual sugarcane stalks (stems) increased by 4.11 times compared to the control group during the same period. |
| Sorghum | The average weight of a single sorghum stalk (stem) increased by 3.46 times compared to the control group during the same period. | The average weight of a single sorghum stalk (stem) increased by 3.52 times compared to the control group during the same period. |
| Rapeseed plant | The average weight of individual Rapeseed plant (stem and leaves) increased by 4.15 times compared to the control group during the same period. | The average weight of individual Rapeseed plant (stem and leaves) increased by 4.23 times compared to the control group during the same period. |
| Lettuce | The average weight of individual lettuce (leaves) increased by 2.70 times compared to the control group during the same period. | The average weight of individual lettuce (leaves) increased by 2.75 times compared to the control group during the same period. |
| Beetroot | The average weight of individual beet plants (stems and leaves) increased by 2.71 times compared to the control group during the same period. | The average weight of individual beet plants (stems and leaves) increased by 2.80 times compared to the control group during the same period. |
| Tobacco | The average weight of a single tobacco leaf increased by 3.63 times compared to the control group during the same period. | The average weight of individual tobacco (leaves) increased by 3.67 times compared to the control group during the same period. |
| Alfalfa | The average weight of individual alfalfa plants (stems and leaves) increased by 2.41 times compared to the control group during the same period. | The average weight of individual alfalfa plants (stems and leaves) increased by 2.68 times compared to the control group during the same period. |
| Rubber Grass | The average weight of single rubber grass (stem) increased by 2.73 times compared to the control group during the same period. | The average weight of individual guayule (stem) increased by 2.80 times compared to the control group during the same period. |
| Cotton | The weight of single cotton bolls (seed cotton) per plant increased by an average of 3.86 times compared to the control group during the same period. | The average weight of cotton bolls (seed cotton) per plant increased by 3.62 times compared to the control group during the same period. |
| Flax | The average weight of individual flax (stems, leaves, seeds) increased by 3.75 times compared to the control group during the same period. | The average weight of individual flax (stems, leaves, seeds) increased by 3.94 times compared to the control group during the same period. |
| Sunflower | The average weight of a single sunflower (fruit) increased by 3.83 times compared to the control group during the same period. | The average weight of a single sunflower (fruit) increased by 3.46 times compared to the control group during the same period. |
| Camelina | The average weight of individual camelina (seeds) increased by 4.03 times compared to the control group during the same period. | The average weight of individual camelina (seeds) increased by 4.01 times compared to the control group during the same period. |
| Nutsedge | The average weight of individual nutsedge (stem) increased by 2.72 times compared to the control group during the same period. | The average weight of individual nutsedge (stem) increased by 2.76 times compared to the control group during the same period. |
| Cannabis | The average weight of a single cannabis plant (stem and leaves) increased by 2.90 times compared to the control group during the same period. | The average weight of a single cannabis plant (stems and leaves) increased by 2.84 times compared to the control group during the same period. |
| Poplar | The average weight of a single poplar (root) increased by 2.00 times compared to the control group during the same period. | The average weight of individual poplar (roots) increased by 2.03 times compared to the control group during the same period. |
| Cassava | The average weight of individual cassava (tuber) increased by 2.71 times compared to the control group during the same period. | The average weight of a single cassava (tuber) increased by 2.78 times compared to the control group during the same period. |

The above Table 6 may use the porcine FTO-polypeptide sequence of Sequence Listing SEQ ID NOs:2 or DNAfor expressing said polypeptide sequence, its functionally equivalent homologous sequences, or its functionally equivalent variants/conjugates.

**Table 7 - Inducing medium derived from Bovine (Bos taurus) FTO (Experimental Group)**

| Plant | The average increase in yield by X multiples. | The average increase in yield by Y multiples. |
|---|---|---|
| Rice | The average weight of individual rice (grains) increased by 3.71 times compared to the control group during the same period. | The average weight of a single rice plant's grain (fruit) increased by 3.92 times compared to the control group during the same period. |
| Potato | The average weight of individual potato (tubers) per plant increased by 3.71 times compared to the control group during the same period. | The average weight of a single potato (tuber) increased by 3.54 times compared to the control group during the same period. |
| Com | The average weight of a single com (kemels) increased by 4.05 times compared to the control group during the same period. | The average weight of a single com (kemels) increased by 3.16 times compared to the control group during the same period. |
| Soybean Plant | The average weight of a single soybean (seed) increased by 3.92 times compared to the control group during the same period. | The average weight of a single soybean (seed) increased by 3.43 times compared to the control group during the same period. |
| Tomato | The average weight of a single tomato (fruit) increased by 3.47 times compared to the control group during the same period. | The average weight of a single tomato (fruit) increased by 3.46 times compared to the control group during the same period. |
| Wheat | The average weight of a single wheat plant (fruit) increased by 3.61 times compared to the control group during the same period. | The average weight of a single wheat plant (fruit) increased by 4.10 times compared to the control group during the same period. |
| Sugarcane | The average weight of individual sugarcane stalks (stems) increased by 3.94 times compared to the control group during the same period. | The average weight of a single sugarcane stalk (stem) increased by 4.03 times compared to the control group during the same period. |
| Sorghum | The average weight of individual sorghum stalks (stems) increased by 3.41 times compared to the control group during the same period. | The average weight of a single sorghum stalk (stem) increased by 3.40 times compared to the control group during the same period. |
| Rapeseed plant | The average weight of individual Rapeseed plant (stems and leaves) increased by 4.13 times compared to the control group during the same period. | The average weight of individual Rapeseed plant (stems and leaves) increased by 4.12 times compared to the control group during the same period. |
| Lettuce | The average weight of individual lettuce (leaves) increased by 2.56 times compared to the control group during the same period. | The average weight of individual lettuce (leaves) increased by 2.58 times compared to the control group during the same period. |
| Beetroot | The average weight of individual beet plants (stems and leaves) increased by 2.51 times compared to the control group during the same period. | The average weight of individual beet (stems and leaves) increased by 2.66 times compared to the control group during the same period. |
| Tobacco | The average weight of a single tobacco (leaf) increased by 3.47 times compared to the control group during the same period. | The average weight of individual tobacco (leaves) increased by 3.49 times compared to the control group during the same period. |
| Alfalfa | The average weight of individual alfalfa plants (stems and leaves) increased by 2.43 times compared to the control group during the same period. | The average weight of individual alfalfa plants (stems and leaves) increased by 2.71 times compared to the control group during the same period. |
| Rubber Grass | The average weight of individual rubber plant (stem) increased by 2.50 times compared to the control group during the same period. | The average weight of individual rubber plant (stem) increased by 2.69 times compared to the control group during the same period. |
| Cotton | The average weight of cotton bolls (seed cotton) per plant increased by 3.67 times compared to the control group during the same period. | The average weight of cotton bolls (seed cotton) per plant increased by 3.42 times compared to the control group during the same period. |
| Flax | The average weight of individual flax (stems, leaves, seeds) increased by 3.55 times compared to the control group during the same period. | The average weight of a single flax plant (stem, leaves, seeds) increased by 3.91 times compared to the control group during the same period. |
| Sunflower | The average weight of a single sunflower (fruit) increased by 3.62 times compared to the control group during the same period. | The average weight of a single sunflower (fruit) increased by 3.37 times compared to the control group during the same period. |
| Camelina | The average weight of individual camelina (seeds) increased by 4.01 times compared to the control group during the same period. | The average weight of individual camelina (seeds) increased by 4.05 times compared to the control group during the same period. |
| Nutsedge | The average weight of a single nutsedge (stem) increased by 2.78 times compared to the control group during the same period. | The average weight of individual nutsedge (stem) increased by 2.80 times compared to the control group during the same period. |
| Cannabis | The average weight of a single cannabis plant (stem and leaves) increased by 2.90 times compared to the control group during the same period. | The average weight of a single cannabis plant (stems and leaves) increased by 2.81 times compared to the control group during the same period. |
| Poplar | The average weight of individual poplar (roots) increased by 2.07 times compared to the control group during the same period. | The average weight of individual poplar (roots) increased by 2.01 times compared to the control group during the same period. |
| Cassava | The average weight of a single cassava (tuber) increased by 2.72 times compared to the control group during the same period. | The average weight of a single cassava (tuber) increased by 2.75 times compared to the control group during the same period. |

The above Table 7 may use the bovine FTO-polypeptide sequence of Sequence Listing SEQ ID NOs:3 or DNA for expressing polypeptide sequences, its functionally equivalent homologous sequences, or its functionally equivalent variants/conjugates.

**Table 8-Inducing medium derived from Green Flagellate Algae (Ostreococcus lucimarinus) FTO (Experimental Group)**

| Plant | The average increase in yield by X multiples. | The average increase in yield by Y multiples. |
|---|---|---|
| Rice | The average weight of individual rice (grains) increased by 3.41 times compared to the control group during the same period. | The average weight of individual rice (grains) increased by 3.46 times compared to the control group during the same period. |
| Potato | The average weight of individual potato (tubers) per plant increased by 3.37 times compared to the control group during the same period. | The average weight of individual potato (tubers) per plant increased by 3.38 times compared to the control group during the same period. |
| Com | The average weight of individual com (kemels) increased by 3.35 times compared to the control group during the same period. | The average weight of a single com (kemels) increased by 3.39 times compared to the control group during the same period. |
| Soybean Plant | The average weight of individual soybean plant (seeds) increased by 3.38 times compared to the control group during the same period. | The average weight of individual soybean plant (seeds) increased by 3.34 times compared to the control group during the same period. |
| Tomato | The average weight of individual tomato fruits increased by 3.43 times compared to the control group during the same period. | The average weight of individual tomato (fruit) increased by 3.42 times compared to the control group during the same period. |
| Wheat | The average weight of a single wheat plant (fruit) increased by 3.15 times compared to the control group during the same period. | The average weight of single wheat (grains) per plant increased by 3.23 times compared to the control group during the same period. |
| Sugarcane | The average weight of individual sugarcane stalks (stems) increased by 3.26 times compared to the control group during the same period. | The average weight of a single sugarcane stalk (stem) increased by 3.29 times compared to the control group during the same period. |
| Sorghum | The average weight of a single sorghum stalk (stem) increased by 3.40 times compared to the control group during the same period. | The average weight of individual sorghum stalks (stems) increased by 3.53 times compared to the control group during the same period. |
| Rapeseed plant | The average weight of a single Rapeseed plant plant (stem and leaves) increased by 3.22 times compared to the control group during the same period. | The average weight of individual Rapeseed plant (stem and leaves) increased by 3.37 times compared to the control group during the same period. |
| Lettuce | The average weight of lettuce (leaves) per plant increased by 3.39 times compared to the control group during the same period. | The average weight of individual lettuce (leaves) increased by 3.41 times compared to the control group during the same period. |
| Beetroot | The average weight of individual beet (stems and leaves) increased by 3.12 times compared to the control group during the same period. | The average weight of individual beet (stems and leaves) increased by 3.09 times compared to the control group during the same period. |
| Tobacco | The average weight of individual tobacco (leaves) increased by 3.37 times compared to the control group during the same period. | The average weight of a single tobacco leaf increased by 3.63 times compared to the control group during the same period. |
| Alfalfa | The average weight of individual alfalfa plants (stems and leaves) increased by 3.37 times compared to the control group during the same period. | The average weight of individual alfalfa plants (stems and leaves) increased by 2.76 times compared to the control group during the same period. |
| Rubber Grass | The average weight of individual guayule (stem) increased by 3.30 times compared to the control group during the same period. | The average weight of individual guayule (stem) increased by 2.80 times compared to the control group during the same period. |
| Cotton | The average weight of single cotton bolls (seed cotton) per plant increased by 3.32 times compared to the control group during the same period. | The average weight of single cotton bolls (seed cotton) increased by 3.60 times compared to the control group during the same period. |
| Flax | The average weight of individual flax (stems, leaves, seeds) increased by 3.06 times compared to the control group during the same period. | The average weight of individual flax (stems, leaves, seeds) increased by 3.81 times compared to the control group during the same period. |
| Sunflower | The average weight of individual sunflower (fruit) increased by 3.43 times compared to the control group during the same period. | The average weight of a single sunflower (fruit) increased by 3.58 times compared to the control group during the same period. |
| Camelina | The average weight of individual camelina (seeds) increased by 3.06 times compared to the control group during the same period. | The average weight of individual camelina (seeds) increased by 3.89 times compared to the control group during the same period. |
| Nutsedge | The average weight of a single nutsedge (stem) increased by 3.31 times compared to the control group during the same period. | The average weight of individual nutsedge (stem) increased by 2.81 times compared to the control group during the same period. |
| Cannabis | The average weight of a single cannabis plant (stems and leaves) increased by 3.12 times compared to the control group during the same period. | The average weight of individual cannabis (stems and leaves) increased by 2.85 times compared to the control group during the same period. |
| Poplar | The average weight of individual poplar (roots) increased by 3.25 times compared to the control group during the same period. | The average weight of individual poplar (roots) increased by 2.09 times compared to the control group during the same period. |
| Cassava | The average weight of single cassava (tuber) increased by 3.32 times compared to the control group during the same period. | The average weight of single cassava (tuber) increased by 2.80 times compared to the control group during the same period. |

The above Table 8 may use the polypeptide sequence of Chlamydomonas reinhardtii FTO listed in Sequence Table SEQ ID NOs:4, or the DNA for expressing the polypeptide sequence, its homologous sequences with equivalent functions, or its variants/conjugates with equivalent functions.

**Table 9-Examples of FTO Introduction into Plants Induced by Media Derived from Various Algae and Invertebrates**

| Introduction Sequence | Plant | The average increase in yield by X multiples. | The average increase in yield by Y multiples. |
|---|---|---|---|
| Aphanomyces astaci | Rice | The average weight of a single rice plant's grain (fruit) increased by 3.18 times compared to the control group during the same period. | The average weight of individual rice (grains) increased by 3.21 times compared to the control group during the same period. |
| Fusion of Micromonas commoda | Potato | The average weight of a single potato (tuber) increased by 3.36 times compared to the control group during the same period. | The average weight of individual potato (tubers) per plant increased by 3.39 times compared to the control group during the same period. |
| Micromonas pusilla | Com | The average weight of individual com (kemels) increased by 3.29 times compared to the control group during the same period. | The average weight of individual com (kemels) increased by 3.20 times compared to the control group during the same period. |
| Dinoflagellate Polarella glacialis | Soybean Plant | The average weight of individual soybean plant (seeds) increased by 3.33 times compared to the control group during the same period. | The average weight of individual soybean plant (seeds) increased by 3.35 times compared to the control group during the same period. |
| Guillardia theta | Tomato | The average weight of individual tomato fruits increased by 3.07 times compared to the control group during the same period. | The average weight of individual tomato fruits increased by 3.17 times compared to the control group during the same period. |
| Ostreococcus tauri | Wheat | The average weight of individual wheat (grains) increased by 3.21 times compared to the control group during the same period. | The average weight of individual wheat plants (fruits) increased by 3.26 times compared to the control group during the same period. |
| Ectocarpus sp. | Sugarcane | The average weight of a single sugarcane stalk (stem) increased by 3.44 times compared to the control group during the same period. | The average weight of a single sugarcane stalk (stem) increased by 3.49 times compared to the control group during the same period. |
| Conferva siliculosa | Sorghum | The average weight of individual sorghum stalks (stems) increased by 3.14 times compared to the control group during the same period. | The average weight of individual sorghum stalks (stems) increased by 3.28 times compared to the control group during the same period. |
| Fragilariopsis cylindrus | Rapeseed plant | The average weight of individual Rapeseed plant (stem and leaves) increased by 3.33 times compared to the control group during the same period. | The average weight of individual Rapeseed plant (stems and leaves) increased by 3.41 times compared to the control group during the same period. |
| Acanthaster planci | Lettuce | The average weight of individual lettuce (leaves) increased by 3.43 times compared to the control group during the same period. | The average weight of individual lettuce (leaves) increased by 3.40 times compared to the control group during the same period. |
| Saccoglossus kowalevskii | Beetroot | The average weight of individual beet plants (stems and leaves) increased by 3.20 times compared to the control group during the same period. | The average weight of individual beet plants (stems and leaves) increased by 3.28 times compared to the control group during the same period. |
| Aphanomyces astaci | Tobacco | The average weight of individual tobacco leaves increased by 3.21 times compared to the control group during the same period. | The average weight of individual tobacco (leaves) increased by 3.27 times compared to the control group during the same period. |
| Fusion of Micromonas commoda | Alfalfa | The average weight of individual alfalfa plants (stems and leaves) increased by 3.11 times compared to the control group during the same period. | The average weight of individual alfalfa plants (stems and leaves) increased by 3.10 times compared to the control group during the same period. |
| Micromonas pusilla | Rubber Grass | The average weight of a single rubber plant (stem) increased by 3.31 times compared to the control group during the same period. | The average weight of individual guayule plants (stems) increased by 3.35 times compared to the control group during the same period. |
| Dinoflagellate Polarella glacialis | Cotton | The average weight of cotton bolls (seed cotton) per plant increased by 3.36 times compared to the control group during the same period. | The average weight of single cotton bolls (seed cotton) per plant increased by 3.32 times compared to the control group during the same period. |
| Guillardia theta | Flax | The average weight of a single flax plant (stem, leaves, seeds) increased by 3.08 times compared to the control group during the same period. | The average weight of individual flax (stems, leaves, seeds) increased by 3.18 times compared to the control group during the same period. |
| Ostreococcus tauri | Sunflower | The average weight of a single sunflower (fruit) increased by 3.37 times compared to the control group during the same period. | The average weight of a single sunflower (fruit) increased by 3.35 times compared to the control group during the same period. |
| Ectocarpus sp. | Camelina | The average weight of individual camelina (seeds) increased by 3.28 times compared to the control group during the same period. | The average weight of individual camelina (seeds) increased by 3.31 times compared to the control group during the same period. |
| Conferva siliculosa | Nutsedge | The average weight of individual nutsedge (stem) increased by 3.22 times compared to the control group during the same period. | The average weight of a single nutsedge (stem) increased by 3.28 times compared to the control group during the same period. |
| Fragilariopsis cylindrus | Cannabis | The average weight of a single cannabis plant (stems and leaves) increased by 3.45 times compared to the control group during the same period. | The average weight of a single cannabis plant (stems and leaves) increased by 3.46 times compared to the control group during the same period. |
| Acanthaster planci | Poplar | The average weight of a single poplar (root) increased by 3.30 times compared to the control group during the same period. | The average weight of individual poplar (roots) increased by 3.35 times compared to the control group during the same period. |
| Saccoglossus kowalevskii | Cassava | The average weight of individual cassava (tubers) increased by 3.27 times compared to the control group during the same period. | The average weight of individual cassava (tubers) increased by 3.21 times compared to the control group during the same period. |

The above Table 9 may use various algal sequences and invertebrate sequences or DNA for expressing those sequences from the Sequence Listing SEQ ID NOs: 5~15, their homologous sequences with equivalent functions, or their variants/conjugates with equivalent functions.

The above is merely a preferred embodiment of the present invention. It should be noted that the aforementioned preferred embodiment should not be considered as a limitation on the present invention. The scope of protection of the present invention shall be defined by the scope of the claims. For those of ordinary skill in the art, several improvements and modifications can be made without departing from the spirit and scope of the present invention, and these improvements and modifications should also be regarded as within the scope of protection of the present invention.
Sequence Listing:
   DTD Version: V1_3
   Filename: 1.xml
   Software Name: WIPO Sequence
   Software Version: 2.3.0
   Date of Generation: 2023-08-30
Basic Information:
   Current Application / Intellectual Property Office: CN
   Current Application / Applicant File Name: 24059CNCNP2-1
   Applicants Name or Designation: 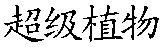 ( 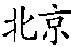 ) 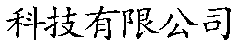
   Applicants Name or Title / Language: zh
   Applicants Name or Designation / Latin Name: EpiPlant CO. LTD.
   Inventor Names: Jia Guifang, Li Ji, Li Weifeng
   Name of Inventor / Language: zh
   Inventor's Name / Latin Name: Jia Guifang, Li Ji, Li Weifeng
   Title of Invention: Method for Cultivating Plants, Application, and Product
   Total Sequence Quantity: 15
Sequence:
   Serial Number (ID): 1
   Length: 505
   Molecular Type: AA
   Feature Location/Qualifier:
      - source, 1..505
         > mol_type, protein
         > organism, Homo sapiens
   Residue: 
MKRTPTAEER EREAKKLRLL EELEDTWLPY LTPKDDEFYQ QWQLKYPKLI LREASSVSEE 60
LHKEVQEAFL TLHKHGCLFR DLVRIQGKDL LTPVSRILIG NPGCTYKYLN TRLFTVPWPV 120
KGSNIKHTEA EIAAACETFL KLNDYLQIET IQALEELAAK EKANEDAVPL CMSADFPRVG 180
MGSSYNGQDE VDIKSRAAYN VTLLNFMDPQ KMPYLKEEPY FGMGKMAVSW HHDENLVDRS 240
AVAVYSYSCE GPEEESEDDS HLEGRDPDIW HVGFKISWDI ETPGLAIPLH QGDCYFMLDD 300
LNATHQHCVL AGSQPRFSST HRVAECSTGT LDYILQRCQL ALQNVCDDVD NDDVSLKSFE 360
PAVLKQGEEI HNEVEFEWLR QFWFQGNRYR KCTDWWCQPM AQLEALWKKM EGVTNAVLHE 420
VKREGLPVEQ RNEILTAILA SLTARQNLRR EWHARCQSRI ARTLPADQKP ECRPYWEKDD 480
ASMPLPFDLT DIVSELRGQL LEAKP 505
Serial Number (ID): 2
Length: 505
Molecular Type: AA
Feature Location/Qualifier:
   - source, 1..505
      > mol_type, protein
      > organism, Sus scrofa
Residue: 
MKRTPTAEER ERGAKKLRLL EELEDTWLPY LTPKDDEFYQ QWQLKYPKLI LREAGSVPEG 60
LHKEVQEAFL ALHKHGCLFR DLVRIQGKDL LTPVSRLLIG NPGCTYKYLN TRLFTVPWPV 120
 
KGSDAKYNEA EIGAACQTFL KLNDYLQIET IQALEELAAK EKANIDTVPA CIGPDFPRVG 180
MGSSFDGHDE VDRKSRAAYN LTLLSFMDPQ KMPYLKEEPY FGMGKMAVSW HHDENLVDRS 240
AVAVYNYSCE GPEEESEDDP QLEGRNPDVW HVGFKISWDI ETPGLAIPLH QGDCYFMLDD 300
LNATHQHCVL AGLPPRFSST HRVAECSTGA LDYILQRCQL ALQNVRDEAD SGEVSLKSLE 360
PAVLKQGEEI HNEVEFEWLR QFWFQGNRYK KCTDWWCQPM TQLEELWKKM EGATHAVLRE 420
VRREGAPVEQ SSDILTAILA VLTTRQNLRR EWHARCQSRI ARTLPVDQKP ECRPYWEKDD 480
PSMPLPFDLT DTVAELRGLL LEAKP 505
Serial Number (ID): 3
Length: 505
Molecular Type: AA
Feature Location/Qualifier:
   - source, 1..505
      > mol_type, protein
      > organism, Bos taurus
Residue: 
MKRTPTAEER EREAKKLRLL EELEDTWLPY LTPKDDEFYQ QWQLKYPKLI LREAASVPEL 60
LHKEVQQAFL TLHKHGCLFR DLVRIQGKDL LTPVSRILIG NPGCTYKYLN TRLFTVPWPV 120
KGSDAKYNEA EIAAACQTFL KLNSYLQVET IQALEELAAK EKANIDAVPV CIGPDFPRVG 180
MGSSFDGHDE IDMKNRAAYN VTLLNFMDPQ KMPYLKEEPY FGMGKMAVSW HHDENLVDRS 240
AVAVYSYSCE GPEEESEDDP QLEGRDPDIW HVGFKISWDI ETPGLAIPLH QGDCYFMLDD 300
LNATHQHCVL AGLPPRFSST HRVAECSTGT LEYILQRCQV ALQNVREEAD NGEISLKSLE 360
SVVLKQGEEI HNEVEFEWLR QFWFQGSRYK KCTDWWCQPM SQLEEMWRKM EWLTSAVLRE 420
VRREGVPMEQ KNEMLTSILA SITTRQNLRR EWHARCQSRI ARTLPADQKP ECRPYWEKGD 480
PSMPLPFDLT EIVSELRGLL LETRP 505
Serial Number (ID): 4
Length: 419
Molecular Type: AA
Feature Location/Qualifier:
   - source, 1..419
      > mol_type, protein
      > organism, Ostreococcus lucimarinus
Residue: 
MSPSSSVLEP EDGEPFARVH RAHYRGFVVD APSVLPASLH DDVERAFDDM RSRGEFTHDV 60
VSAGNKVSTT YVRRCLLGED GMTYHYQKLR LFAQPWRGRE AYEVVRRLNE TLTRSARERC 120
EKLGGAFAES ECEYNVTLIN YMETEGESEI ELRNEEKFDL GTTSVSWHSD SSLRENSTVA 180
VYHTYEAPER KDWRVALRAL NAECEVLCVP LEDKATYYMC GEFNATHHHA VLTGSSARYS 240
STHRVAVVAK DTFQYIKRRC IDALAIVPDL ERENKPLDAK QIQFLADVHR EVEFQWIRMF 300
HLQGEAHAAW HDTYWTRKIA ELTEAWDRME ACFRVILSKL KRSSRSPESP PRAYAMLLYA 360
 LKTVKELRDE YTKRTKASAY ASLPPSQRPV DLPAYDNTSP LPFELKPVIY FLEEEQNKV 419
Serial Number (ID): 5
Length: 522
Molecular Type: AA
Feature Location/Qualifier:
   - source, 1..522
      > mol_type, protein
      > organism, Aphanomyces astaci
Residue: 
MDPKERKKLE RQKKKAKALE RKSAKKPNVA SLKPVSQPTT SHKRPSEPSG SSAAPPAKKQ 60
QIEKAKPLPV PSNYTGPEKF LTPSHPDFHD ILTRSYVGFV HEPAAAQPRE FHAGMRAAFE 120
HLRDKGYFQY DVVMAGGKHL SRTFVRRTLV GNFGITYKYL GLRIFAHAWD VPSSAAVFRN 180
LYALNQAIVA RTKASASPAP PSCEYNLTLI NYMEPDNPVL GLKDDLTYDM GKVSVSWHAD 240
SSLEDFSSIG VYHTLLPTKK SKKGPCDWRI AMRLSPDVPG AKTTPPLVVP TGDGDVYYLN 300
GDFNHHHQHM VLSGTAVRVS STHRVAVTAE DTLQYIQAKV TAALKSSAKD PQDKCRRGHA 360
DDVRHEQTTV TTLEMDWLRQ YWIQGDKHND LHVDWQQPML ALEASWRKLE ARTKTLADAL 420
VQSHDVDDLK VLKAVLEGLR NRQVGRDQFV HRMKDKVFKR IAADFQPMER PVWTDWDDVH 480
LLPKDLTATI AALHAHKLVL ESEKKRQWKI HAGTRHNKIN KA 522
Serial Number (ID): 6
Length: 520
Molecular Type: AA
Feature Location/Qualifier:
   - source, 1..520
      > mol_type, protein
      > organism, Micromonas commode
Residue: 
MGKKHKPRPD DRARVVDASD VGGKVKKKKA NGATMKASRR KGGGASANGD GRPYDPSRTL 60
SKPAPPPPAY TPRVPTGAYI TPEHDEYDAC VARSYVGFVV DPPDILPDEL HADVAAAFKT 120
MAKRGLFTRD VLAAGKTVSP TFVSRTLVGE RGMTYHYQKL RIFALPWSDE DTPEGSPLRV 180
VRRLNDAMKR RSRALLTRHR DARVPGDVTG SCEFNVTLIN LMEPERKESV ALKDEGQFGM 240
GKASVSWHSD SSLQDTSTVA VYHQTSGGID GDDTSWHVAL KCLDGVTPAL RVPLRSHSAY 300
YIMRDFNLHH HHAVLNGDTR RFSSTHRVAV TAKDTFDYVR GRCERALAVL PSLKWRAGRW 360
PKGTKPPRDA SSTQLSDAVR VLGETHREVE FQWIRMFWLQ GTRHAAQHDG YWTRRIEELT 420
QAWDVMELGL RFAMDACARA ADGKAKAEPR AFEMLRYLLE TIAELREEHD ARTQSGAYAQ 480
VPDDCKPVDL PGFDDSSPLP RNLRPVLNTL ERHRRRALDR 520
Serial Number (ID): 7
Length: 541
Molecular Type: AA
Feature Location/Qualifier:
   - source, 1..541
      > mol_type, protein
      > organism, Micromonas pusilla
Residue: 
MATATGSSGG KLEKRKKKKK KTTAVERAGS GGGGNAKKPT TTTASHRPTK PLLRAAPPPP 60
SYRLRGTSGS DDDDDDAAPS FLTPSHAEYE RCMKTSYLGF HVDDPSALPE KTHREIARAF 120
ERMASDGIFH RDVLAAGKSV SPTFVSRTLL GERGMTYHYQ RLRIFAHPWD EETQPDASHP 180
FRAVKRLNDA LVSRSTAVLR ASSDPRLGDG VDGSCDFNVT LINLMEPASR CAEVPLKDEA 240
LFGMGKARST SVSWHSDSSL QDVSTVAVYH QTDDDDDDSW AVALKTLDGV TPAMRTPLRS 300
REMYYMLRDF NAHHHHAVIT GESRRFSSTH RVAVVAKDTF EHIKTRCERA LRLLPGIKKI 360
AQVGPGPGDD LAAFARSAQM MGETHRECEF QWIRMFWLQG SRHAASHSAY WRQRIAELTQ 420
AWDAMELGYR WALDALRAGA STGAMPLRAY DVVSYLLEET RELRAEHARR SRAGAYEAMP 480
ADCRPVDEPK FDEKSPLPFD LGPVLATLAG WRRDAERAQT TTTEGGSKTK KGKPGKGSKR 540
R 541
Serial Number (ID): 8
Length: 506
Molecular Type: AA
Feature Location/Qualifier:
   - source, 1..506
      > mol_type, protein
      > organism, Polarella glacialis
Residue: 
MDMDRFVREP ADASSESFHE EFRRCLHGLY EQGIFQYDLI QPVGPGTPVV STRVTRCLIG 60
EPGITYKYLG LRMFAHPWKG EKTSESCSAM YQHNQELISK AEEQAVAVGC DAPPYSSCFN 120
LTLINRMDPA DSGAPNKLGA VSVSWHADSS LVDYSTITVY VAEHDATQDS FGLSPARPCK 180
PCDDPWRVAL RHTPNVQGPE KSCPWEVAAQ ETAKPVKRRA KAKVHTVPIH DGDIYHLIGD 240
FNHHNQHAVL AGRDAVLAVR RGMPVLGGSG TIRYSSTHRV ALTEGHSYRS VKQRCSTALS 300
VALADMIPAA AQVHEEQQCS LELEFEWIRQ FFIQGKAHRE ALPWWHSKMD ELVFLWRQFE 360
VLTAQRLTLL RRAAAGKVGT SELDPFCLAL KNGLQKRAEL REAWRQREHD PLLKKQPANR 420
QPLQCLFDAP SDAPHGAGSE ALPHNVWPLV EEISQIQLQL HETKKVGKPW YQFVLNVYQG 480
CAFQGGEVTC LSWTLFSQRL PMKRRW 506
Serial Number (ID): 9
Length: 543
Molecular Type: AA
Feature Location/Qualifier:
   - source, 1..543
      > mol_type, protein
      > organism, Guillardia theta
Residue: 
MQETLWQRVK EGLEWFDGAI GEGEEEEEAG RIAGNKGGGR RRGFEQALER YYPKFRVIHR 60
DLLPSALEPS TRRALEVLKD QMGWFRRDYV WVKGKVTQTM VTRLLVGERG CTYKYKGLRL 120
FCRSWETEAA KVPDSACHAL AEIRRLNEVL KEKSRKQHEC FMARGEGSGE GAVEGGVNSF 180
NATLINYFDE SIANHVETRP EPYYGMGRCC ISWHADDGVV EQSAIAVYNV IERAEKGEEE 240
ELGEVWKVGL KVKWEVDTPG VAVPMVNGDA YVMLSGFNEK NQHCVVCGRG SRFSSTHRVC 300
PQEDMYHNFY CRCRETFLQL HFPWNARGVL QEGEERRACL SKIVASLDAL EDLHDQLEFE 360
WLRQFHFQYF DLPPSELDDY WVEVIHVLAD LWQRMEIVVW IWISCCRRKS FMVGGKELRD 420
RLERLRQLTS QQKGQGQEVQ SSLPFLMKKL LRSEGAGGGR EAAGEKAIPE DALELVKATL 480
ARRLQKRQEH RIWWEENFRK RLSMGRGGEQ REGGKRKRKE GRADGLGFSL LPANLNSLVS 540
TLK 543
Serial Number (ID): 10
Length: 420
Molecular Type: AA
Feature Location/Qualifier:
   - source, 1..420
      > mol_type, protein
      > organism, Ostreococcus tauri
Residue: 
MPDVLGPGDG DAYERVRSFH YDGFVVDDPE ILPEALHDDV ARAFDSLLRE GEFTHDVLSA 60
GNKVSRTFVT RTLLGDRGTT YHYQKLRLFA QPWDGEKRWE YATARRLNEA LTRRTREILR 120
GTRHGESECE YNVTLINRMD TREESEVALK DEALFDLGTT SVSWHSDSSL RENSTVAVYS 180
TVDDPERADW SVALRALGKQ CPALRVPLKD KATYYMCGDF NATHHHAVLT GSSARYSSTH 240
RVGVVEKDTF QYIKRRCLEA LALIPDLENK SASLDAKKIQ LLAEIHREVE FQWIRMFHLQ 300
GDTHARQHKT YWTQRIAELT EAWDRMEACF RAILSKLKLS PESTPQPSRA YSMMLYAMKE 360
VKELRDEHVK RISASAYAAL PESQRPVDLP QYDDTSPLPF ELKPVIYFVE AEQAKLKGES 420
Serial Number (ID): 11
Length: 787
Molecular Type: AA
Feature Location/Qualifier:
   - source, 1..787
      > mol_type, protein
      > organism, Ectocarpus sp
Residue: 
MTMTTATTVA TTSRLLSPPT SEATTPIQHS NSGMEVSTEK TSHKKKRKSK RKRQQQERDT 60
SSIPDASRQS RGSSSSSTRP PPPPPPPPPT TTTTTTSKKH SRIKEKPARF VHPHLDAELR 120
 
RLGRSWLSPG DPLFKPALDR SYRGVRHEPG GSLPQAVHRG FRQSFDSLHE AGLFLYDTVQ 180
AGGKRLSRTF VTRTLVGDAG ITYKYLGLRL FAHPWGLGES DFRKGPGDSL ADALNDPCLA 240
DLFSNDGSGF DDNDRVADTL RKVGDLSEWM RLRAGAMLKE EVGEAGGERG SCAFNLTLIN 300
RMECSGAKRD LKPDPLFSMG KFSVSWHADS CLQDFSTIGV YHCTDPVPAE PDWGIALRVS 360
TEARVGGGGG GSGDGDGYGV SNVEKTRRKE AERGVKPAGP KAIASVAASA AGAVPEKQGE 420
QVLTPPLLIP LQSGDAYFLL DDFNHFHEHA VVSGSSTLRY SSTHRVADTD GNTFSHVQKR 480
CERALAGGGS AAAGGDYHIL NLAQVRAEQA LLTELESEWL NQFWVQGRRH ASLHPWWRGP 540
MALVLATWRE LERRTALTVR RLMSLRGQGG GEGVGPGGGG TAAETAGAEP AAIAKAFETV 600
LRALEVRRES RSRWGARFAD SAYRSLAADL APVVPALWPA EGSSGGDVGE EGSPLLLPQS 660
LGGIVSRLRE CYTVVYRAAS GGKKAGSAKA IAAGAASNWE LFRAASSSSS SGGGGAGKKH 720
GSALGKQQQQ QQQQQQQQRK MRGLAGQDGL EKTKKRAGGV VKGGGDGGGG RTLKGQGSRR 780
KDRVRER 787
Serial Number (ID): 12
Length: 715
Molecular Type: AA
Feature Location/Qualifier:
   - source, 1..715
      > mol_type, protein
      > organism, Conferva siliculosa
Residue: 
MTTTSATTAA RSRLPSPPTS AATTPIQHSN NGTEASPEKT SNKKKAKNKR KRQQECSIPA 60
ASQQSRGSSS SSTRPPPPTT TTLKKHSRIK EKPARFVHPH LDAELRRLGR SWLSPGDPLF 120
TPALDRSYRG VRHEPAGSLP QAVHSGFRQS FDSLHEAGLF LYDTVQAGGK RLSRTFVTRT 180
LVGDAGITYK YLGLRLFAHP WGLGESDFRK GPGDGLADAL NDPCLADLFS KDGASSVRKS 240
KDNLRGSRFD DNDRVADTLR KVGDLSEWMR LRAGAMLKEE VREAGGERGS CAFNLTLINR 300
MECSGAKRDL KPDPLFSMGK CSVSWHADSC LQDFSTIGVY HCTDPVPAEP DWGIALRVST 360
EARVGGDGGG DGDGNGVSND EKTRRKEAER GLKPAGPKAV ASVAASAAEA VPEKQGEQVL 420
TPPLLIPLQS GDAYFLLDDF NHFHEHAVVS GSSTLRYSST HRVADTDGNT FSHVQKRCEQ 480
ALAGGGSAAA GGDDQILNLA QVRAEQALLT ELESEWLNQF WVQGRRHASL HPWWRGPMAL 540
VLATWRELER RTSLTVQHLM SLRGQDGGEA LWPAEGSGGG DVGEEGSTLL LPQSLGGVIS 600
RLRECYTVVY RAASGGKKAG SAKAIAAGAA SNWELFRAAS SSASSGGGGA GKKHGSALGK 660
QQQQQQQRKK RGAAGQDGLE KKKRAGGVVK GGGDGGGGRT SKGQRSRRKG RVRER 715
Serial Number (ID): 13
Length: 417
Molecular Type: AA
Feature Location/Qualifier:
   - source, 1..417
      > mol_type, protein
      > organism, Fragilariopsis cylindrus
Residue: 
MLTPEDGVYF QKHLQDHYKG FVHLPVDRLE PRDFHEEFKN SLERLRDAGY YQYDVVMAGG 60
KYSSRTFVKR TLVGNPGITY KYLGLRLFAH AWSGPGCTPL MKSIGDMNQS MIKMTEQFPE 120
NERCDYNLTL INYMEPTTHT KVGFKDEANY GMGKVSVSWH ADSSLVDNSS IGVYHCLPTQ 180
RNSKWDWKIA LRPLAIESKD NTGSTKRDNK STTPKPVVVN TKDGDAYFLL GNFNMKHQHC 240
VLAGSQANRI SSTHRVAVTI EDTYDYILKR VKIARKRFRL QMETIPPSLQ SPVVGLDAKV 300
IRYCQRILTE VEMEWIAQYW LQGDQHDKMR VWWQKPMKTL EAYWCALEVY TYRLFEFLLS 360
RIASNDTVPI DVVKVLIIEF KTRQSFREQW DERRSDKIYQ RRVSEEFRPV ARPIFEN 417
Serial Number (ID): 14
Length: 528
Molecular Type: AA
Feature Location/Qualifier:
   - source, 1..528
      > mol_type, protein
      > organism, Acanthaster planci
Residue: 
MRFLSLNVLR RWMSKMSKRR QTEDLGKEVK KKKLLSEVAR GRLQFLMPDH PDFKSLMRKS 60
YFNFAHEPST GIPVSLHEEL TWALDILRYH GYFYRDWVKV KGQTVQTPIS RTLIGEPGTT 120
YKYLGLRLFT IPWSFGHHKP ESEVELASET IGKLNEHYKA VSRRLLRKKT AEQTHLKSSP 180
SQEVAPCSSK DRPRERICVE EPKQMTHGLS IEEGTNFNVV LINYMDPEDP DLRLKPEPYY 240
GMGPLAVSWH MDGNLVRGST VAVYNHTCSS DGGDSTTDSD WMVGFKVSWD IETPGVAIPL 300
QTGESYFMLG DLNDTHQHCV VAGSQARFST THRVADCQQG TLSYIQQRCL EALGNLSQLE 360
NGEYELKSFQ AGHLDFTETV HNEVEFEWLR QFWMQGSRHA AERGGWVPAM ECLEADWNKM 420
EHMTKLAVEE ANRLGPEEGS VIAEALLPHL KERRKLRQEC LDKFPRRLLE TIHRDFLPIH 480
RPKWDNDDPS QPLPFDLKGI IRKMRLLCNG KRIKSPSDSE SSDTGLHS 528
Serial Number (ID): 15
Length: 485
Molecular Type: AA
Feature Location/Qualifier:
   - source, 1..485
      > mol_type, protein
      > organism, Saccoglossus kowalevskii
Residue: 
MNPRMKRKIP RIPLVTSEGE VAGQLALPIR KQKLLSAVKE TQVKFLSPVD TDYKALVAEN 60
YDNFVVQPGT ELSSELHTRL QQGFETLLTN GYLYQHRIRF RNKTVMTPVS RTLIGEPGMT 120
YRYLNLRLFA LPWTFDDKKK LSDVEKACNA FYELNTYFKD HVNKLITEKF ASSTGRKIDE 180
 
TDDSLKTIIQ RNTEFKATIL NYMDPSEIRL KQEPYYNMGD IAVSWHKDES LVDGSTVAVY 240
NHVPMEARQD CSKWKVGLKV AWDITTPAVC TPLNNKDCYY MLDTMNDTHQ HCVIAGTLPR 300
YSSTHRVVES SDSTLCYIQQ RCQTALDNIG YKDQIPYLKS LETDVLQLIE EIHSEIEFEW 360
LRQFWMQGTQ HSQERQYWLK PMEQLQEDWK TMELMTKLMI DIVVKKKDQS HMIAKILLPS 420
LVERNELRNE WMNRYSEKEF YHIPEEHRPI YEPCWTDANS EMPLPFDLSQ HIDIIQECMD 480
TNDSQ 485
END

## Claims

1. A plant cultivation method, **characterized by** introducing an inducing medium into the initial plant and obtaining a target plant through cultivating which contains no inducing medium and has optimized traits compared to the initial plant, wherein the inducing medium can induce demethylation of 6-methylated bases in plant RNA.

2. The plant cultivation method according to claim 1, **characterized in that** the target plant has at least one trait optimization compared to the initial plant, with an optimization degree Y.

3. The plant cultivation method according to claim 1, **characterized in that** an inducing medium is transiently introduced into the initial plant, directly obtaining the target plant which contains no inducing medium and has optimized traits compared to the initial plant.

4. The plant cultivation method according to claim 1, **characterized in that** an inducing medium is transiently introduced into the protoplasts of the initial plant.

5. The plant cultivation method according to claim 1, **characterized in that** an inducing medium is introduced into the initial plant to obtain an intermediate plant, which is subjected to progeny cultivating to obtain a target plant which contains no inducing medium and has optimized traits compared to the initial plant.

6. The plant cultivation method according to claim 5, **characterized in that** the intermediate plant contains an inducing medium.

7. The plant cultivation method according to claim 5, **characterized in that** the target plant has at least one trait optimization compared to the initial plant, with an optimization degree Y.

8. The plant cultivation method according to claim 7, **characterized in that** the intermediate plant has at least one trait optimization compared to the initial plant, with an optimization degree X.

9. The plant cultivation method according to any one of claims 2 or 7, **characterized in that** Y is an increase ratio compared to the initial plant at the same period, Y≥0.20; Y≥0.50; Y≥1.00; preferably Y≥2.00; preferably Y≥3.00; preferably Y≥4.00.

10. The plant cultivation method according to claim 1, **characterized in that** the trait optimization includes yield increase.

11. The plant cultivation method according to claim 1, **characterized in that** the trait optimization includes an increase in the yield of plant organs.

12. The plant cultivation method according to claim 1, **characterized in that** the trait optimization includes at least one increase with respect to the volume of plant organs, quantity, weight, or the number of tillers.

13. The plant cultivation method according to claim 1, **characterized in that** an inducing medium can induce the demethylation of 6-methyladenine in plant RNA.

14. The plant cultivation method according to claim 1, **characterized in that**, an inducing medium is introduced in an amount excessive for the initial plant.

15. The plant cultivation method according to claim 1, **characterized in that** the inducing medium is nucleic acid molecule and/or polypeptide, its homolog, its functional variant or conjugate.

16. The plant cultivation method according to claim 1, **characterized in that** when the inducing medium is nucleic acid molecule, its homolog, its functional variant, or its conjugate, the target plant does not contain inducing medium, nor does it contain polypeptide obtained from expression of inducing medium; when inducing medium is polypeptide, its homolog, its functional variant, or its conjugate, the target plant does not contain inducing medium.

17. The plant cultivation method according to claim 1, **characterized in that** the inducing medium is selected from m6A demethylase of RNA and its encoding nucleic acid.

18. The plant cultivation method according to claim 1, **characterized in that** the inducing medium is selected from at least one of FTO nucleic acid molecule and/or polypeptide, its homolog, its functional variant or conjugate.

19. The plant cultivation method according to claim 1, **characterized in that** the FTO in the inducing medium is derived from vertebrates, invertebrates, algae, their orthologs, or their paralogs.

20. The plant cultivation method according to claim 1, **characterized in that** the inducing medium is as shown in the Sequence Listing Seq.ID.No1~Seq.ID.No15.

21. The plant cultivation method according to claim 1, **characterized in that** the initial plant is selected from at least one of food crops, feed crops, fiber crops, oil crops, sugar crops, beverage crops, spice crops, condiment crops, medicinal crops, dye crops, ornamental crops, fruit crops, and vegetable crops; preferably selected from at least one of rapeseed plant, tomato, lettuce, and beet; or preferably selected from at least one of rice, com, soybean plant, potato, wheat, millet, sugarcane, sorghum, and cassava; or preferably selected from at least one of tobacco, alfalfa, rubber grass, cotton, flax, sunflower, camelina, nutsedge, cannabis, and poplar.

22. The plant cultivation method according to claim 1, **characterized in that** the initial plant part, which is subjected to introducing treatment, includes at least one of plant organ, plant tissue, and plant cell.

23. The plant cultivation method according to claim 1, **characterized in that** initial plant part, which is subjected to introducing treatment, is selected from meristematic tissues.

24. The plant cultivation method according to claim 1, **characterized in that** an inducing medium is introduced into at least one of the nucleus and cytoplasm of the initial plant.

25. The plant cultivation method according to claim 1, **characterized in that** the introduction method includes introducing into the initial plant a vector carrying an inducing medium.

26. The plant cultivation method according to claim 1, **characterized in that** the progeny cultivation method includes at least one of natural genetic screening removal, hybrid genetic screening removal, and active removal.

27. The use of the plant cultivation method according to claim 1 for the preparation of plants having optimized traits and free of the inducing medium.

28. A product derived from the target plant obtained by the plant cultivation method of claim 1 and subjected to inactivation treatment.
